(19) **Europäisches Patentamt European Patent Office Office européen des brevets**

(11) **EP 3 871 720 B1**

(12) # EUROPEAN PATENT SPECIFICATION

(45) Date of publication and mention
of the grant of the patent:
**20.03.2024 Bulletin 2024/12**

(21) Application number: **19877300.4**

(22) Date of filing: **24.10.2019**

(51) International Patent Classification (IPC):
***A61M 16/00*** *(2006.01)* ***A61M 16/12*** *(2006.01)*
***A61M 16/16*** *(2006.01)* ***A61M 16/20*** *(2006.01)*
***A61M 16/06*** *(2006.01)*

(52) Cooperative Patent Classification (CPC):
(C-Sets available)
**A61M 16/0677; A61M 16/0069; A61M 16/024;**
**A61M 16/125; A61M 16/204;** A61M 16/16;
A61M 2016/0021; A61M 2016/0027;
A61M 2202/0208; A61M 2205/13; A61M 2205/3334

(Cont.)

(86) International application number:
**PCT/CN2019/113125**

(87) International publication number:
**WO 2020/083350 (30.04.2020 Gazette 2020/18)**

(54) **VENTILATION THERAPY APPARATUS**

BEATMUNGSTHERAPIEVORRICHTUNG

APPAREIL DE THÉRAPIE DE VENTILATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.10.2018 CN 201811261598**

(43) Date of publication of application:
**01.09.2021 Bulletin 2021/35**

(73) Proprietor: **BMC Medical Co., Ltd.**
**Shijingshan**
**Beijing 100041 (CN)**

(72) Inventors:
• **ZHAN, Muxia**
**Beijing 100041 (CN)**
• **WANG, Qingsong**
**Beijing 100041 (CN)**
• **ZHUANG, Zhi**
**Beijing 100041 (CN)**

(74) Representative: **Grünecker Patent- und**
**Rechtsanwälte**
**PartG mbB**
**Leopoldstraße 4**
**80802 München (DE)**

(56) References cited:
**WO-A1-2017/055995      KR-A- 20110 094 649**
**US-A1- 2016 287 824**

(52) Cooperative Patent Classification (CPC): (Cont.)

    C-Sets
    A61M 2202/0208, A61M 2202/0007

## Description

CROSS REFERENCE TO RELEVANT APPLICATIONS

The present disclosure claims the priority of the Chinese patent application filed by State Intellectual Property Office of The P.R.C on October 26th, 2018 with the application number of 201811261598.4, and the title of "VENTILATION THERAPY APPARATUS AND CONTROL METHOD FOR SAME".

TECHNICAL FIELD

The present disclosure relates to the medical equipment field and, more particularly, to a ventilation therapy apparatus and a method for controlling the ventilation therapy apparatus.

BACKGROUND

In modern clinical medicine, ventilation therapy apparatuses play a very important role in the field of modern medicine. Ventilation therapy apparatus is a vital medical apparatus that can prevent and treat respiratory failure, reduce complications, and save and prolong the lives of patients, which may mix pure oxygen with air and provide to the patients. Ventilation therapy apparatus are shown in US 2016/287824 A1, KR 2011 0094649 A and WO 2017/055995 A1.

At present, common ventilation therapy apparatuses are usually divided into two types, which includes a wearable respiratory mask and a plug-in nasal oxygen tube ventilation therapy apparatus. For the first type of ventilation therapy apparatuses, the wearable respiratory mask may form an enclosed space on the patient side and supply air to the enclosed space for the patient to breathe, excess air may be exhausted through the vent on the mask. Before using, the patient may adjust the flow of pure oxygen input into the ventilation therapy apparatus, so that the oxygen and the fixed flow of air output by the ventilation therapy apparatus are mixed in advance to form a mixed air flow with fixed oxygen concentration. For the second type of ventilation therapy apparatus, the plug-in nasal oxygen tube ventilation therapy apparatus may keep the airway open by inserting the end of the soft and deformable nasal oxygen tube into the patient's nasal cavity, so that the nasal oxygen tube may supply air to the patient's nasal cavity directly, and excess air may be directly exhausted through the gap of the patient's nasal cavity.

However, in the conventional technology, the amount of oxygen output by the wearable respiratory mask remains constant during work. If a total flow or an oxygen concentration needs to be changed during the oxygen supply process, users can only turn off the ventilation therapy apparatus and re-adjust the flow of oxygen and air, resulting inconvenience to use. While the plug-in nasal oxygen tube is an open air path, and it is difficult to monitor the output flow in real time through the sensor, which makes it impossible to change the total flow or the oxygen concentration at any time during the oxygen supply process.

SUMMARY

The invention is defined by the appended claims. Subject-matter referred as embodiments and/or disclosures which are not claimed are not part of the invention.

The present disclosure provides a ventilation therapy apparatus and a method for controlling the ventilation therapy apparatus, to solve the problem that the ventilation therapy apparatus in the prior art cannot change the total flow or the oxygen concentration at any time during the oxygen supply process.

In order to solve the above technical problem, the present disclosure is realized as follows:

In the first aspect, a ventilation therapy apparatus according to the present invention is defined in claim 1.

The embodiment of the present disclosure uses the feedback of the output parameters output by the main body to determine the patient's expiratory and inspiratory flow, and correspondingly adjusts the opening degree of the oxygen proportional valve and the rotating speed of the fan of the main body to deliver the airflow corresponding to the patient's respiratory flow or pressure to the patient, so that the patient may receive a constant concentration oxygen more comfortably, and improve the patient's respiratory experience.

The output parameters comprise: an airflow pressure and an air flow at the output end of the main body.

Optionally,

the control module is configured for calculating an airflow pressure at the patient interface through the airflow pressure and the air flow; and
when the airflow pressure at the patient interface is not equal to a first preset target pressure value, it is determined that a current working state of the main body is a use state, the control module is configured for controlling the oxygen proportional valve to open at the corresponding preset opening degree, and controlling the fan of the main body to run at the corresponding preset rotating speed.

In the embodiment of the present disclosure, the airflow pressure at the patient interface may be calculated by the airflow pressure and the air flow, the first preset target pressure value is a pressure at the patient interface in an ideal state. When the airflow pressure at the patient interface is not equal to a first preset target pressure value, it is accurately determined the current working state of the main body is the use state. In the use state, control the oxygen proportional valve to open at the corresponding preset opening degree, and control the fan of the main body to run at the corresponding preset rotating speed, which may not only supply on demand, but also avoid waste.

Optionally, the operation that when the airflow pressure at the patient interface is not equal to a first preset target pressure value, it is determined that a current working state of the main body is a use state, the control module is configured for controlling the oxygen proportional valve to open at the corresponding preset opening degree, and controlling the fan of the main body to run at the corresponding preset rotating speed, comprises:

if the airflow pressure at the patient interface is less than the first preset target pressure value, it is determined that the current working state of the main body is an inspiratory state, the control module further is configured for controlling the oxygen proportional valve to open at a corresponding second opening degree, and controlling the fan of the main body to run at a corresponding second preset rotating speed; and

if the airflow pressure at the patient interface is larger than the first preset target pressure value, it is determined that the current working state of the main body is an exhalation state, the control module is further configured for controlling the oxygen proportional valve to open at a corresponding third opening degree, and controlling the fan of the main body to run at a corresponding third preset rotating speed.

In the embodiment of the present disclosure, the first preset target pressure value is a pressure value in the ideal state without interference from pressure drop. When the patient is inhaling, the main body mixes more oxygen by inhaling with more air inhaled by the fan, so that the flow output by the ventilation therapy apparatus is slightly larger than the patient's inhalation flow, while maintaining a constant oxygen concentration for auxiliary inhalation; when it is determined the patient is exhaling, the main body mixes less oxygen by inhaling with less air inhaled by the fan, so that the ventilation therapy apparatus outputs a small flow rate while maintaining a constant oxygen concentration, to prevent the patient's exhaled air from flowing back to the ventilation therapy apparatus. The embodiment of the present disclosure controls the oxygen proportional valve to open at different opening degrees by the control module according to different breathing states, so that the corresponding flow oxygen may be continuously provided with the patient's breathing, which reduces the escape of oxygen into the outside air and avoids waste, moreover, by mixing the oxygen with the air inhaled by the fan, the oxygen content in the gas mixture may be kept constant. When the patient is inhaling, the output of the ventilation therapy apparatus is slightly larger than the patient's inhalation flow; when the patient is exhaling, the ventilation therapy apparatus outputs a small flow rate to prevent the patient's exhaled gas from flowing back to the ventilation therapy apparatus, and provides the patient with a constant concentration of oxygen, which is convenient for the doctor to formulate the treatment plan and confirm the treatment effect.

Optionally, when the airflow pressure at the patient interface is equal to a second preset target pressure value and is maintained for a preset time, it is determined that the current working state of the main body is a non-use state, the control module is further configured for controlling the oxygen proportional valve to open at a corresponding first opening degree, and controlling the fan of the main body to run at a corresponding first preset rotating speed; wherein the second preset target pressure value is less than the first preset target pressure value.

In the embodiment of the present disclosure, the second preset target pressure value may be a pressure value close to 0, when the airflow pressure at the patient interface is equal to the second preset target pressure value, and it is maintained for the preset time, it shows that at this moment the patient does not use the ventilation therapy apparatus, it is a standby mode, the patient interface exposes to the air, the control module controls the oxygen proportional valve to open at the corresponding first opening degree, and meanwhile controls the fan of the main body to run at the corresponding first preset rotating speed, to continually output a small pressure and flow, which may ensure the temperature and the humidity inside the respiratory pipe to be constant.

Optionally, the second opening degree is larger than the first opening degree, the first opening degree is larger than or equal to the third opening degree; the second preset rotating speed is larger than the first preset rotating speed, and the first preset rotating speed is larger than or equal to the third preset rotating speed.

In the embodiment of the present disclosure, when the patient is inhaling, the main body mixes more oxygen by inhaling with more air inhaled by the fan, so that the flow output by the ventilation therapy apparatus is slightly larger than the patient's inhalation flow, while maintaining a constant oxygen concentration for auxiliary inhalation; when it is determined the patient is exhaling, the main body mixes less oxygen by inhaling with less air inhaled by the fan, so that the ventilation therapy apparatus outputs a small flow rate while maintaining a constant oxygen concentration, to prevent the patient's exhaled air from flowing back to the ventilation therapy apparatus. In the standby mode, the patient interface exposes to the air, continually outputs a small pressure and flow, which may ensure the temperature and the humidity inside the respiratory pipe are constant. So that the corresponding flow oxygen may be continuously provided with the patient's breathing, which reduces the escape of oxygen into the outside air and avoids waste, moreover, by mixing the oxygen with the air inhaled by the fan, the oxygen content in the gas mixture may be kept constant.

Optionally, the ventilation therapy apparatus further comprises: a humidifier, configured for heating and humidifying the output gas.

In the embodiment of the present disclosure, the gas provided by the main body is heated and humidified by the humidifier, so that it may meet the breathing needs of the user and improve the breathing effect.

Optionally, when the airflow pressure at the patient interface is equal to a second preset target pressure value and is maintained for a preset time, it is determined that the current working state of the main body is a standby state, the control module is further configured for controlling the oxygen proportional valve to close, and controlling the humidifier to start, and controlling the fan of the main body to run at a corresponding fourth preset rotating speed; wherein the fourth preset rotating speed is less than the first preset rotating speed.

In the embodiment of the present disclosure, when the current working state of the main body is the standby state, the fan runs at the lower fourth preset speed to output a very small air flow, the oxygen proportional valve is closed, and the humidifier works normally to maintain the temperature and provide normal temperature and humidity output at any time.

Optionally, the operation that the control module is configured for detecting output parameters of the main body, when it is determined that the main body is in a preset state, the control module controls the oxygen proportional valve to open at a corresponding preset opening degree according to the output parameters, and controls the fan of the main body to run at a corresponding preset rotating speed, comprises:

the control module is configured for detecting the output parameters of the main body; and the control module is further configured for monitoring a respiratory flow value of the patient;
when it is determined that the main body is in a flow priority state, the control module is configured for adjusting the output parameters of the main body to be larger than the respiratory flow value of the patient; and
when it is determined that the main body is in a pressure priority state, the control module is configured for adjusting the output parameters of the main body to be a positive pressure value.

In the embodiment of the present disclosure, when the flow is prioritized, the respiratory flow value may be calculated at any time, to ensure the output flow value of the ventilation therapy apparatus is larger than the patient's respiratory flow value, so as to ensure that all the gas inhaled by the patient is provided by the ventilation therapy apparatus and will not inhale air through the gap between the patient interface and the nasal cavity. When the pressure is prioritized, it is configured to adjust the output parameter of the main body to a positive pressure value, which may ensure that the positive pressure in the nasal cavity is maintained, so as to ensure that the human body does not directly inhale external air.

Optionally, the respiratory pipe and the main body are connected through a gas path and a circuit, and the circuit and the gas path are on and off simultaneously.

In the embodiment of the present disclosure, the respiratory pipe and the main body are connected through the gas path and the circuit, and the circuit and the gas path are on and off simultaneously, which may according to the control signal of the main body, control the circuit and the gas path timely and accurately. At this time, the main body may receive a corresponding state signal timely, and may achieve timely and on-demand supply, and avoid waste.

In the second aspect of the invention, a computer program is provided as defined in claim 10, the computer program comprising a computer readable code, when the computer readable code is run on a computing processing device of a control module of a ventilation therapy apparatus, causing the computing processing device to execute a method for controlling the ventilation therapy apparatus .

In the embodiment of the present disclosure, the patient's inhalation flow and exhalation flow are determined by the feedback of the output parameters output by the main body, and correspondingly adjust the opening degree of the oxygen proportional valve and the rotating speed of the fan of the main body, to deliver the airflow corresponding to the patient's breathing flow or pressure for the patient, so that the patient may receive a constant concentration of oxygen more comfortably, and improve the user's breathing experience.

The output parameters comprise: an airflow pressure and an air flow at an output end of the main body;

Optionally,

the step of when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air, comprises:

calculating an airflow pressure at a patient interface through the airflow pressure and the air flow; and
when the airflow pressure at the patient interface is not equal to a first preset target pressure value, determining that a current working state of the main body is a use state, controlling the oxygen proportional valve to open at the corresponding preset opening degree, and controlling the fan of the main body to run at the corresponding preset rotating speed.

In the embodiment of the present disclosure, the airflow pressure at the patient interface is accurately calculated through the airflow pressure and the air flow, and the first preset target pressure value is the pressure at the patient interface in an ideal state. When the airflow pressure at the patient interface is not equal to the first preset target pressure value, it is accurately determined that the current working state of the main body is the use state. In the use state, the oxygen proportional valve is controlled to open at the corresponding preset opening degree, and at the same time, the fan of the main body is controlled to run at the corresponding preset speed, which may not only supply on demand, but also avoid waste.

Optionally, the step of when the airflow pressure at the patient interface is not equal to a first preset target pressure value, determining that a current working state of the main body is a use state, controlling the oxygen proportional valve to open at the corresponding preset opening degree, and controlling the fan of the main body to run at the corresponding preset rotating speed, comprises:

if the airflow pressure at the patient interface is less than the first preset target pressure value, determining that the current working state of the main body is an inspiratory state, controlling the oxygen proportional valve to open at a corresponding second opening degree, and controlling the fan of the main body to run at a corresponding second preset rotating speed; and
if the airflow pressure at the patient interface is larger than the first preset target pressure value, determining that the current working state of the main body is an exhalation state, controlling the oxygen proportional valve to open at a corresponding third opening degree, and controlling the fan of the main body to run at a corresponding third preset rotating speed.

In the embodiment of the present disclosure, the first preset target pressure value is a pressure value in the ideal state without interference from pressure drop. When the patient is inhaling, the main body mixes more oxygen by inhaling with more air inhaled by the fan, so that the flow output by the ventilation therapy apparatus is slightly larger than the patient's inhalation flow, while maintaining a constant oxygen concentration for auxiliary inhalation; when it is determined the patient is exhaling, the main body mixes less oxygen by inhaling with less air inhaled by the fan, so that the ventilation therapy apparatus outputs a small flow rate while maintaining a constant oxygen concentration, to prevent the patient's exhaled air from flowing back to the ventilation therapy apparatus. The embodiment of the present disclosure controls the oxygen proportional valve to open at different opening degrees by the control module according to different breathing states, so that the corresponding flow oxygen may be continuously provided with the patient's breathing, which reduces the escape of oxygen into the outside air and avoids waste, moreover, by mixing the oxygen with the air inhaled by the fan, the oxygen content in the gas mixture may be kept constant. When the patient is inhaling, the output of the ventilation therapy apparatus is slightly larger than the patient's inhalation flow; when the patient is exhaling, the ventilation therapy apparatus outputs a small flow rate, to prevent the patient's exhaled gas from flowing back to the ventilation therapy apparatus, and provides the patient with a constant concentration of oxygen, which is convenient for the doctor to formulate the treatment plan and confirm the treatment effect.

Optionally, the method further comprises:

when the airflow pressure at the patient interface is equal to a second preset target pressure value and is maintained for a preset time, determining that the current working state of the main body is a non-use state, controlling the oxygen proportional valve to open at a corresponding first opening degree, and controlling the fan of the main body to run at a corresponding first preset rotating speed;
wherein the second preset target pressure value is less than the first preset target pressure value.

In the embodiment of the present disclosure, the second preset target pressure value may be a pressure value close to 0, when the airflow pressure at the patient interface is equal to the second preset target pressure value, and it is maintained for the preset time, it shows that at this moment the patient does not use the ventilation therapy apparatus, it is a standby mode, the patient interface exposes to the air, the control module controls the oxygen proportional valve to open at the corresponding first opening degree, and meanwhile controls the fan of the main body to run at the corresponding first preset rotating speed, to continually output a small pressure and flow, which may ensure the temperature and the humidity inside the respiratory pipe to be constant.

Optionally, the second opening degree is larger than the first opening degree, the first opening degree is larger than or equal to the third opening degree; the second preset rotating speed is larger than the first preset rotating speed, and the first preset rotating speed is larger than or equal to the third preset rotating speed.

In the embodiment of the present disclosure, when the patient is inhaling, the main body mixes more oxygen by inhaling with more air inhaled by the fan, so that the flow output by the ventilation therapy apparatus is slightly larger than the patient's inhalation flow, while maintaining a constant oxygen concentration for auxiliary inhalation; when it is determined the patient is exhaling, the main body mixes less oxygen by inhaling with less air inhaled by the fan, so that the ventilation therapy apparatus outputs a small flow rate while maintaining a constant oxygen concentration, to prevent the patient's exhaled air from flowing back to the ventilation therapy apparatus. In the standby mode, the patient interface exposes to the air, continually outputs a small pressure and flow, which may ensure the temperature and the humidity inside the respiratory pipe are constant. So that the corresponding flow oxygen may be continuously provided with the patient's breathing, which reduces the escape of oxygen into the outside air and avoids waste, moreover, by mixing the oxygen with the air inhaled by the fan, the oxygen content in the gas mixture may be kept constant.

Optionally, the method executed by the computing processing device further comprises

when the airflow pressure at the patient interface is equal to the second preset target pressure value and is maintained for the preset time, determining that the current working state of the main body is a standby state, controlling the oxygen proportional valve to close, and controlling a humidifier to start, and controlling the fan of the main body to run at a corresponding fourth preset rotating speed;
wherein the fourth preset rotating speed is less than the first preset rotating speed.

In the embodiment of the present disclosure, when the current working state of the main body is the standby state, the fan runs at the lower fourth preset speed, to output a very small air flow, the oxygen proportional valve is closed, and the humidifier works normally, to maintain the temperature and provide normal temperature and humidity output at any time.

Optionally, the step of when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air, comprises:

monitoring a respiratory flow value of the patient;
when it is determined that the main body is in a flow priority state, adjusting the output parameters of the main body to be larger than the respiratory flow value of the patient; and
when it is determined that the main body is in a pressure priority state, adjusting the output parameters of the main body to be a positive pressure value.

In the embodiment of the present disclosure, when the flow is prioritized, the respiratory flow value may be calculated at any time, to ensure the output flow value of the ventilation therapy apparatus is larger than the patient's respiratory flow value, so as to ensure that all the gas inhaled by the patient is provided by the ventilation therapy apparatus, and will not inhale air through the gap between the patient interface and the nasal cavity. When the pressure is prioritized, it is configured to adjust the output parameter of the main body to a positive pressure value, which may ensure that the positive pressure in the nasal cavity is maintained, so as to ensure that the human body does not directly inhale external air.

The ventilation therapy apparatus according to the embodiment of the present disclosure, includes: the main body, the respiratory pipe, the patient interface, the oxygen supply module, the oxygen proportional valve and the control module; the main body is configured for outputting gas with a preset pressure and a preset flow, and the main body comprises an output end; the respiratory pipe comprises a first end and a second end which communicates with each other, and the first end of the respiratory pipe communicates with the output end; the second end of the respiratory pipe is connected to the patient interface, the patient interface is configured for being worn on a patient's nasal cavity, when the patient interface is worn on the patient's nasal cavity, an air outlet gap is disposed between the patient interface and the patient's nasal cavity; the oxygen supply module is connected to the main body through the oxygen proportional valve, the control module is configured for detecting output parameters of the main body, and when it is determined that the main body is in a preset state, the control module controls the oxygen proportional valve to open at a corresponding preset opening degree according to the output parameters, and controls the fan of the main body to run at a corresponding preset rotating speed. The present disclosure uses the feedback of the output parameters output by the main body to determine the patient's expiratory and inspiratory flow, and adjusts the opening degree of the oxygen proportional valve and the rotating speed of the fan of the main body, to deliver the airflow corresponding to the patient's respiratory flow or pressure to the patient, so that the patient may receive a constant concentration oxygen more comfortably, and improve the user's respiratory experience.

Described above is merely an overview of the inventive scheme. In order to more apparently understand the technical means of the disclosure to implement in accordance with the contents of specification, and to more readily understand above and other objectives, features and advantages of the disclosure, specific embodiments of the disclosure are provided hereinafter.

BRIEF DESCRIPTION OF THE DRAWINGS

In order to more clearly explain the technical solutions in the embodiments of the present disclosure or the prior art, the following will briefly introduce the drawings that need to be used in the description of the embodiments or the prior art. Obviously, the drawings in the following description are some embodiments of the present disclosure. For those of ordinary skill in the art, other drawings may be obtained based on these drawings without creative work.

Fig. 1 is a structural schematic diagram of the ventilation therapy apparatus according to an embodiment of the present disclosure;
Fig. 2 is a structural schematic diagram of the main body and the respiratory pipe according to an embodiment of the present disclosure;
Fig. 3 is a schematic diagram showing a flow-time of the patient's respiratory process according to an embodiment of the present disclosure;
Fig. 4 is a schematic diagram showing a flow-pressure of the patient's respiratory process according to an embodiment of the present disclosure;
Fig. 5 is another schematic diagram showing a flow-time of the patient's respiratory process according to an embodiment of the present disclosure;
Fig. 6 is a schematic diagram showing a pressure-time of the patient's respiratory process according to an embodiment of the present disclosure;
Fig. 7 is a flow chart of the method executed by the computing processing device for controlling the ventilation therapy apparatus ;
Fig. 8 is a specific flow chart of the method executed by the computing processing device for controlling the ventilation therapy apparatus ;
Fig. 9 is a computing processing device that can implement the method according to the present disclosure ; and
Fig. 10 is a portable or fixed storage module according to an example of the present disclosure

DETAILED DESCRIPTION

In order to make the purpose, technical solutions and advantages of the embodiments of the present disclosure clearer, the technical solutions in the embodiments of the present disclosure will be described clearly and completely in conjunction with the accompanying drawings in the embodiments of the present disclosure. Obviously, the described embodiments is a part of the embodiments of the present disclosure, but not all of the embodiments. Based on the embodiments in the present disclosure, all other embodiments obtained by those of ordinary skill in the art without creative work shall fall within the protection scope of the present disclosure.

The following describes in detail the ventilation therapy apparatus and the method for controlling the ventilation therapy apparatus according to the present disclosure by listing several specific embodiments.

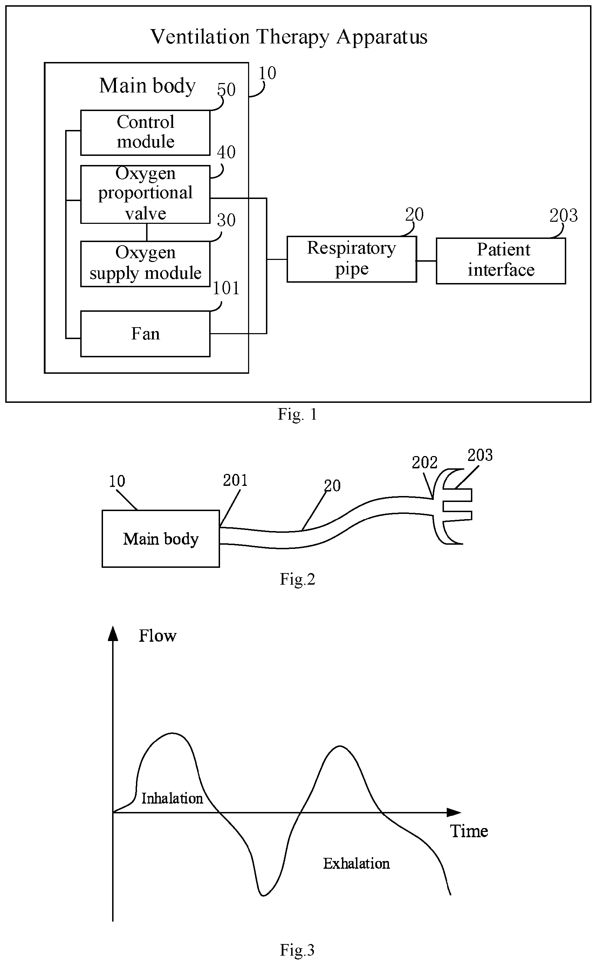

Referring to Fig. 1, there is shown a structural block diagram of the ventilation therapy apparatus according to the present disclosure, the ventilation therapy apparatus comprises: a main body 10, a respiratory pipe 20, a patient interface 203, an oxygen supply module 30, an oxygen proportional valve 40 and a control module 50, the main body 10 is configured for outputting gas with a preset pressure and a preset flow, and the main body 10 comprises an output end, the oxygen supply module 30 is connected to the main body 10 through the oxygen proportional valve 40; the control module 50 is connected to the main body 10 and the oxygen proportional valve 40, respectively.

Preferably, referring to Fig. 2, it is shown a structural schematic diagram of the main body and the respiratory pipe according to the present disclosure, the respiratory pipe 20 comprises a first end 201 and a second end 202 which communicates with each other, and the first end 201 of the respiratory pipe 20 communicates with the output end of the main body 10; the second end 202 of the respiratory pipe 20 is connected to the patient interface 203, the patient interface 203 is configured for being worn on a patient's nasal cavity, when the patient interface 203 is worn on the patient's nasal cavity, an air outlet gap is disposed between the patient interface 203 and the patient's nasal cavity.

In addition, referring to Fig. 2, the structural of the patient interface 203 includes two branch tubes divided at the end, which deliver gas to the two nostrils of the patient separately. An inner diameter of the branch tube at the end of the patient interface 203 is larger than 4 mm, a length is larger than 4 mm, and a thinnest part of a tube wall is less than 0.5 mm. Because these two branch tubes are not an oxygen suction tubes, the flow rate of the oxygen suction tube is usually only 5 to 15 liters per minute, however, these two branch tubes need a large enough airflow (more than 60 liters per minute (LPM)) to generate the required positive pressure, so their output flow is relatively large, so the inner diameter is larger than an inner diameter of the conventional oxygen suction tube. But if an outer diameter of the branch tube at the end is also thick, it will hinder the nostril exhalation, and touching an inner wall will make the patient feel uncomfortable. Therefore, the tube wall should be as thin as possible, and not take up an exhaust area of the nostril.

The control module 50 is configured for detecting output parameters of the main body 10, and when it is determined that the main body 10 is in a preset state, the control module controls the oxygen proportional valve 40 to open at a corresponding preset opening degree according to the output parameters, and controls the fan 101 of the main body 10 to run at a corresponding preset rotating speed.

In the embodiment of the present disclosure, the ventilation therapy apparatus includes the main body 10 that provides air, the fan 101 may be disposed inside the main body 10, the fan 101 may be connected to the control module 50, after receiving a control command from the control module 50, the fan 101 rotates at a corresponding preset speed, to make the outside air to be sucked into the main body 10. In addition, the oxygen supply module 30 may be connected to the main body 10 through the oxygen proportional valve 40. The oxygen proportional valve 40 may receive a control signal from the control module 50, and change its opening degree according to the control signal, to adjust the oxygen flow rate provided by the oxygen supply module 30 to the main body 10, the main body 10 mixes the inhaled air with the oxygen provided by the oxygen supply module 30, and provides the gas mixture to the patient through the respiratory pipe 20.

In practical application, referring to Fig. 3, it is shown a schematic diagram showing a flow-time of the patient's respiratory process according to the present disclosure, when the patient uses the ventilation therapy apparatus to breath, the total flow of his inhaled gas will change with time. Therefore, in order to optimize the patient's breathing experience, two different levels of positive pressures may be provided to the patient during the patient's exhalation and inhalation process. When the patient is inhaling, it provides a larger pressure and a larger flow rate, which facilitates the inhalation of more air, and provides a smaller pressure and a smaller flow rate when exhaling, to avoid blockage of the patient's airway. At the same time, when providing a larger pressure and a larger flow rate or a smaller pressure and a smaller flow rate, the volume of air inhaled by the main body 10 and the volume of oxygen provided by the oxygen supply module 30 need to be adjusted accordingly, to ensure the oxygen concentration in the gas inhaled by the patient is constant, but, in the current wearable breathing mask, because the oxygen volume has been disposed by the patient before using the breathing mask, so that a fixed flow of oxygen is provided, and the oxygen volume cannot be changed during use, resulting in the output flow cannot be changed with the patient's respiratory process, so the patient's breathing experience cannot be optimized.

Preferably, referring to Fig. 2, the respiratory pipe 20 used in the embodiment of the present disclosure may be a common nasal oxygen tube in the current medical equipment field, the second end 202 of the respiratory pipe 20 may be provided with a nasal congestion, the nasal congestion extends into the patient's nasal cavity, to form an open air path for oxygen supply. Excess gas may be directly discharged through the gap of the patient's nasal cavity, the environment outside the nasal cavity is the atmospheric pressure.

Therefore, in the embodiment of the present disclosure, the main body 10 may output an output parameter to the patient through the respiratory pipe 20, and calculate the airflow pressure at the patient interface according to the feedback of the output parameter, and identify the patient's exhalation and inhalation flow according to the change of the airflow pressure at the patient interface, which realizes the monitoring of flow changes during the patient's breathing, to allow the main body 10 realizes real-time adjustment of the output flow and the pressure, and keeping the output flow slightly larger than the patient's inhalation flow or keeping a positive pressure in the patient's nasal cavity all the time.

At this time, during oxygen supply, by adjusting the opening degree of the oxygen proportional valve 40, it may realize the real-time adjustment of the oxygen flow provided by the oxygen supply module 30, which may maintain the output oxygen concentration is constant. Hence, the embodiment of the present disclosure may ensure that the patient receives a stable concentration of oxygen more comfortably, by delivering an airflow with a flow or pressure slightly higher than the patient's breathing flow or pressure to the patient.

For example, when the patient is inhaling, the ventilation therapy apparatus outputs a flow slightly larger than the patient's inhalation volume for auxiliary inhalation; when the patient is exhaling, the ventilation therapy apparatus outputs a small flow, to prevent the patient's exhaled gas from flowing back to the ventilation therapy apparatus; or, when the patient is inhaling, the patient's respiratory tract is under negative pressure, and the ventilation therapy apparatus maintains the output pressure is a positive pressure; when the patient is exhaling, the output pressure of the ventilation therapy apparatus is slightly higher than the patient's exhalation pressure, to prevent the patient's exhaled gas from flowing back to the ventilation therapy apparatus.

In summary, the ventilation therapy apparatus according to the embodiment of the present disclosure, includes: the main body, the respiratory pipe, the patient interface, the oxygen supply module, the oxygen proportional valve and the control module; the main body is configured for outputting gas with a preset pressure and a preset flow, and the main body comprises an output end; the respiratory pipe comprises a first end and a second end which communicates with each other, and the first end of the respiratory pipe communicates with the output end; the second end of the respiratory pipe is connected to the patient interface, the patient interface is configured for being worn on a patient's nasal cavity, when the patient interface is worn on the patient's nasal cavity, an air outlet gap is disposed between the patient interface and the patient's nasal cavity; the oxygen supply module is connected to the main body through the oxygen proportional valve, the control module is configured for detecting output parameters of the main body, and when it is determined that the main body is in a preset state, the control module controls the oxygen proportional valve to open at a corresponding preset opening degree according to the output parameters, and controls the fan of the main body to run at a corresponding preset rotating speed. The present disclosure uses the feedback of the output parameters output by the main body to determine the patient's expiratory and inspiratory flow, and adjusts the opening degree of the oxygen proportional valve and the rotating speed of the fan of the main body, to deliver the airflow corresponding to the patient's respiratory flow or pressure to the patient, so that the patient may receive a constant concentration oxygen more comfortably, and improve the user's respiratory experience.

Optionally, the output parameters comprise: an airflow pressure and an air flow at the output end of the main body; the control module 50 is configured for calculating an airflow pressure at the patient interface through the airflow pressure and the air flow; and when the airflow pressure at the patient interface is not equal to a first preset target pressure value, it is determined that a current working state of the main body 10 is a use state, the control module 50 is configured for controlling the oxygen proportional valve 40 to open at the corresponding preset opening degree, and controlling the fan 101 of the main body 10 to run at the corresponding preset rotating speed.

In the embodiment of the present disclosure, the air flow characteristics in the respiratory pipe 20 are certain, according to an energy equation of a fluid, a steady flow of the incompressible fluid in the tube has the following formula:

$$\frac{U^2}{2} + \frac{p}{\rho} + e + \Pi = const$$

Wherein, U is a flow rate of the fluid, p is a pressure of the fluid, $\rho$ is a density of the fluid, e is an internal energy of the fluid, $\Pi$ is a potential energy, and const is a constant, which means that in a fluid system, such as airflow and water flow, the faster the flow rate, the less the pressure generated by the fluid.

In addition, an air flow resistance has the following formula:

$$F = \frac{1}{2} C \rho S U^2$$

Wherein, F is an air resistance, $\rho$ is the density of the fluid, C is a resistance coefficient, S is a windward area.

Therefore, combining the inferences derived from the above two formulas, after an incompressible fluid flows through a tube with a length of L and a lateral area of S at a certain flow rate, the pressure changes as follows:

$$\Delta P = P_1 - P_2 = \rho(e_2 - e_1) = \rho \times F \times L = \frac{1}{2} C \rho^2 S U^2 L \propto U^2$$

That is, the pressure drop of the incompressible fluid (the pressure drop is a value of the pressure $P_1$ at the first end 201 of the respiratory pipe 20 minus the pressure $P_2$ at the patient interface) and the flow rate are quadratic. However, the gas is a compressible fluid, so when the pressure drops, the density $\rho$ will increase slightly.

Therefore, when the patient is breathing, it will produce changes in airflow, so that it will cause a pressure drop between the first end of the respiratory pipe and the end of the patient interface. The flow of the gas passing through the respiratory pipe and the pressure drop $\Delta P$ between the first end of the respiratory pipe and the end of the patient interface have a functional relationship $\Delta P = k \ast Flow^n$, wherein n is slightly less than 2. The pressures $P_1$ of the first end of the respiratory pipe and the pressure $P_2$ of the end of the patient interface have a relationship of $P_1 = \Delta P + P_2$. Specifically, k and n may be constants, and the values of k and n may be measured by experiments on the pipe.

Specifically, the experiments on the pipe may include: operating the main body and the oxygen supply module, and placing the patient interface in the air, at this time, the pressure $P_2$ at the patient interface is 0. According to the formula $P_1=\Delta P+P_2$, $\Delta P=P_1$ may be obtained. Recording the flow value and the pressure drop value, multiple experiments, obtaining a linear graph of the flow and the pressure drop. According to the linear graph, the value of k and n may be obtained.

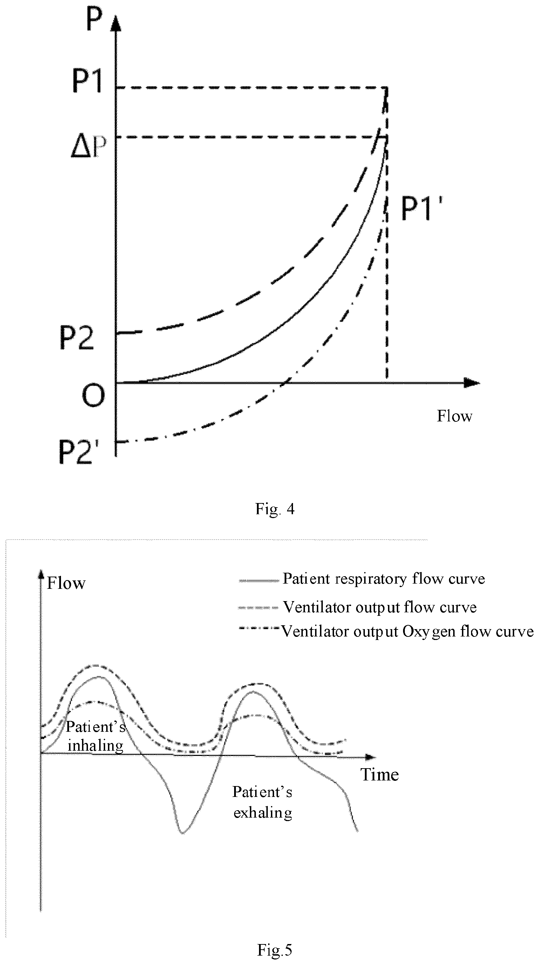

Referring to Fig. 4, it is shown a schematic diagram showing a flow-pressure of the patient's respiratory process according to the present disclosure, when the ventilation therapy apparatus is officially working, the patient interface is inserted into the patient's nasal cavity, and the control module detects the airflow pressure $P_1$ and the flow $F_0$ outputted by the main body itself to the first end of the respiratory pipe, according to the flow-pressure drop linear graph, the theoretical output flow $F_t$ may be calculated from $P_1$, or the pressure drop $\Delta P$ of the air flow through the pipe may be found from $F_0$, and according to the formula $P_1= \Delta P+P_2$, the value of the airflow pressure $P_2$ at the patient interface may be obtained.

In the embodiment of the present disclosure, before the patient uses the ventilation therapy apparatus, a first preset target pressure value will be preset according to their own conditions, and the first preset target pressure value is the pressure at the patient interface in an ideal state. According to the airflow pressure $P_1$ and the airflow flow $F_0$ outputted by the main body 10, the actual pressure $P_2$ at the patient interface may be calculated. Through real-time monitoring of $P_2$ and comparing $P_2$ with the first preset target pressure value $P_t$, it may be determined which preset state the main body is in, that is the exhalation state or the inhalation state, and according to the current state of the main body, the oxygen proportional valve is controlled to open at the corresponding preset opening degree, and at the same time the fan of the main body is controlled to run at the corresponding preset rotating speed, for example, when it is determined that the patient is inhaling, the ventilation therapy apparatus outputs a flow that is slightly larger than the patient's inhalation volume for auxiliary inhalation; when the patient is determined to exhale, the ventilation therapy apparatus outputs a smaller flow rate, to prevent the patient's exhaled gas from flowing back to the ventilation therapy apparatus.

Optionally, if the airflow pressure $P_2$ at the patient interface is less than the first preset target pressure value $P_t$, it is determined that the current working state of the main body 10 is an inspiratory state, the control module 50 is further configured for controlling the oxygen proportional valve 40 to open at a corresponding second opening degree, and controlling the fan 101 of the main body 10 to run at a corresponding second preset rotating speed. If the airflow pressure $P_2$ at the patient interface is larger than the first preset target pressure value $P_t$, it is determined that the current working state of the main body 10 is an exhalation state, the control module 50 is further configured for controlling the oxygen proportional valve 40 to open at a corresponding third opening degree, and controlling the fan 101 of the main body 10 to run at a corresponding third preset rotating speed.

In the embodiment of the present disclosure, before the patient uses the ventilation therapy apparatus, the first preset target pressure value $P_t$ will be preset according to their own conditions, and the first preset target pressure value $P_t$ is the pressure value in an ideal state free from the interference of the pressure drop. When the patient uses the ventilation therapy apparatus, due to the interference of the pressure drop, which will cause the actual pressure $P_2$ at the patient interface is different from the first preset target pressure value $P_t$, and the influence of this pressure drop on the actual pressure $P_2$ at the patient interface may be determined by the comparison result between $P_2$ and $P_t$. Generally, if the actual pressure $P_2$ at the patient interface is less than the first preset target pressure value $P_t$, it is determined that the current working state of the main body 10 is the inhalation state, if the actual pressure $P_2$ at the patient interface is larger than the first preset target pressure value $P_t$, it is determined that the current working state of the main body 10 is the exhalation state.

If it is determined that the current working state of the main body 10 is an inspiratory state, the control module 50 controls the oxygen proportional valve 40 to open at the corresponding second opening degree, and controls the fan 101 of the main body 10 to run at the corresponding second preset rotating speed. If it is determined that the current working state of the main body 10 is an exhalation state, the control module 50 controls the oxygen proportional valve 40 to open at the corresponding third opening degree, and controls the fan 101 of the main body 10 to run at the corresponding third preset rotating speed. Wherein, the second opening degree is larger than the third opening degree, and the second preset rotating speed is larger than the third preset rotating speed, the meaning is that when the patient is inhaling, the main body 10 mixes more inhaled oxygen provided by the oxygen supply module 30 with more air inhaled by the fan 101, so that the ventilation therapy apparatus outputs the flow that is slightly larger than the patient's inhalation volume. While keeping the oxygen concentration constant for auxiliary inhalation; when determining the patient is exhaling, the main body 10 mixes less inhaled oxygen provided by the oxygen supply module 30 with less air inhaled by the fan 101, so that the ventilation therapy apparatus outputs a small flow rate, while maintaining a constant oxygen concentration, so as to prevent the patient's exhaled gas from flowing back to the ventilation therapy apparatus.

Referring to Fig. 5, it is shown a schematic diagram showing a flow-time of the patient's respiratory process according to the present disclosure, which displays the patient's respiratory curve, the output flow curve of the ventilation therapy apparatus, and the output oxygen flow curve of the ventilation therapy apparatus when the patient inhales and exhales. It may be seen that when the patient is inhaling, the total flow and the oxygen flow output by the ventilation therapy apparatus are both larger than the total flow and the oxygen flow rate output by the ventilation therapy apparatus when the patient is exhaling, and because the embodiment of the present disclosure controls the oxygen proportional valve to open at different opening degrees by the control module, according to different breathing states, this makes it possible to continuously provide oxygen at the corresponding flow rate as the patient breathes, reduces the escape of oxygen into the outside air, avoids waste, and mixing the oxygen with the air drawn by the fan may keep the oxygen content in the gas mixture constant. When the patient is inhaling, the output flow of the ventilation therapy apparatus is slightly larger than the patient's inspiratory volume; when the patient is exhaling, the ventilation therapy apparatus outputs a smaller flow, to prevent the patient's exhaled gas from flowing back to the ventilation therapy apparatus, and provides the patient with a constant concentration of oxygen, which is convenient for doctors to formulate treatment plan and confirm the treatment effect.

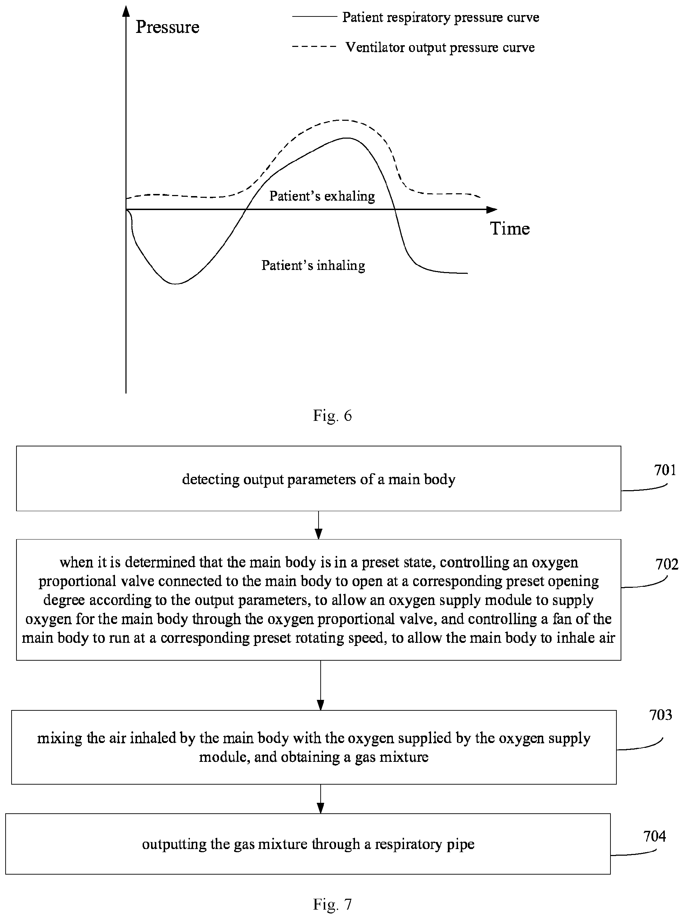

Referring to Fig. 6, it is shown a schematic diagram showing a pressure-time of the patient's respiratory process according to the present disclosure, wherein, the pressure output by the ventilation therapy apparatus is larger than the patient's respiratory pressure, so as to maintain a positive pressure output to the patient's nasal cavity. When the patient is inhaling, the patient's respiratory tract is under negative pressure, and the ventilation therapy apparatus maintains the output pressure is a positive pressure; when the patient is exhaling, the output pressure of the ventilation therapy apparatus is slightly higher than the patient's exhalation pressure, to prevent the patient's exhaled gas from flowing back to the ventilation therapy apparatus. In the embodiment of the present disclosure, it ensures the open air path of the respiratory pipe to realize continuous positive pressure output, and the patient will not inhale air from the external environment.

Specifically, when the airflow pressure $P_2 < 0$ at the patient interface, it may be determined that the patient's nasal cavity is under negative pressure at this time, and the air in the environment will flow into the patient's nasal cavity from the gap between the patient's nasal cavity and the respiratory pipe 20, that is, the patient will inhale a mixture of the ambient air and the gas output by the main body 10; when $P_2 > 0$, the pressure in the patient's nasal cavity is positive pressure, and the airflow is sucked into the patient's airway from the nasal cavity, or flows out from the gap between the nasal cavity and the nasal oxygen tube, and the gas inhaled by the patient at this time is all provided by the main body 10, and will not inhale the air in the environment. At this time, according to the oxygen concentration parameter n disposed by the patient and the total flow $F_0$ output by the main body 10 or air flow $F_{air}$, the required oxygen flow $F_{O2}$ may be calculated, and the quantitative relationship satisfied among the various parameters is as follows:

$$F0 \times n = F_{air} \times 21\% + F_{O2} \times 100\%$$

$$F0 = F_{air} + F_{O2}$$

Therefore, the control module 50 may adjust the opening degree of the oxygen proportional valve 40 by changing the voltage output to the oxygen proportional valve 40, and then adjust the flow of pure oxygen, to ensure that the oxygen concentration of the output gas is a fixed value.

In addition, in another implementation, the size relationship of the patient's respiratory flow and the output flow of the ventilation therapy apparatus may be obtained from $F=F_t-F_0$ ($F_t$ is the theoretical output flow calculated according to $P_1$), if $F > 0$, it means that the flow rate output by the ventilation therapy apparatus is larger than the patient's respiratory flow, at this time, the patient is in the state of exhalation, or in the state of inhalation, and the inhalation volume is all provided by the ventilation therapy apparatus; if $F < 0$, it means that the output flow of the ventilation therapy apparatus is less than the patient's inspiratory flow, and the patient will inhale some air from the environment. At this time, the patient's inhaled oxygen concentration cannot reach the disposed value. Therefore, if $F>0$ may be maintained during the operation of the ventilation therapy apparatus, and the relationship between the oxygen flow $F_{O2}$ and the total flow $F_0$ or the air flow $F_{air}$ is maintained in the relationship of the above equation, it may be ensured that the patient inhales the fixed oxygen concentration gas provided by the ventilation therapy apparatus.

Optionally, when the airflow pressure at the patient interface is equal to a second preset target pressure value and is maintained for a preset time, it is determined that the current working state of the main body 10 is a non-use state, the control module 50 is further configured for controlling the oxygen proportional valve 40 to open at a corresponding first opening degree, and controlling the fan 101 of the main body 10 to run at a corresponding first preset rotating speed; wherein the second preset target pressure value is less than the first preset target pressure value.

In practical application, when the patient wears the nasal oxygen tube, because of its own air resistance, the patient interface will also have a certain pressure due to the patient's air resistance when not breathing. When the nasal oxygen tube is removed, the patient interface is directly connected to the environment, the actual pressure $P_2$ is close

to 0. Because the respiratory tract is negative pressure when inhaling, if the inhalation is strong, the pressure $P_2$ at the patient interface may drop to 0 or a negative value, but it will not be maintained for a long time, the pressure $P_2$ at the patient interface is determined to be close to 0 for a long time, it may be regarded as a non-use state.

Therefore, in the embodiment of the present disclosure, when the pressure $P_2$ at the patient interface is close to 0, it is in the standby state, the second preset target pressure value may be a pressure close to 0, when the airflow pressure at the patient interface is equal to the second preset target pressure value, and maintained for the preset time, it means that the patient does not use the ventilation therapy apparatus, and it is the standby state, the patient interface is exposed to the air, at this time, the control module 50 may control the oxygen proportional valve 40 to open at the corresponding first opening degree, control the fan 101 of the main body 10 to run at the corresponding first preset rotating speed, continually output a small pressure and flow, and ensure the temperature and the humidity inside the respiratory pipe 20 are constant.

Optionally, the second opening degree is larger than the first opening degree, the first opening degree is larger than or equal to the third opening degree; the second preset rotating speed is larger than the first preset rotating speed, the first preset rotating speed is larger than or equal to the third preset rotating speed.

Wherein, the first opening degree may be equal to the third opening degree; the first preset rotating speed may also be equal to the third preset rotating speed. As long as it is ensured that both the first opening degree and the third opening degree are less than the second opening degree; the first preset rotating speed and the third preset rotating speed are both less than the second preset rotating speed.

Optionally, the ventilation therapy apparatus further comprises: a humidifier, configured for heating and humidifying the output gas.

In practical application, the gas people breathe has a certain amount of moisture, and the breathed gas has the highest breathing comfort at a certain temperature. Therefore, the gas provided by the main body may be heated and humidified through the humidifier, so that it may meet the user's breathing needs and improve the breathing effect.

Optionally, when the airflow pressure at the patient interface is equal to the second preset target pressure value and is maintained for the preset time, it is determined that the current working state of the main body is the standby state, the control module is further configured for controlling the oxygen proportional valve to close, and controlling the humidifier to start, and controlling the fan of the main body to run at a corresponding fourth preset rotating speed; wherein the fourth preset rotating speed is less than the first preset rotating speed.

In the embodiment of the present disclosure, when the airflow pressure at the interface of the patient is equal to the second preset target pressure value, and it is maintained for the preset time, it is determined that the current working state of the main body is the standby state. At this time, it is specifically the state that the ventilation therapy apparatus is not connected to the patient after starting, or the patient removes the patient interface and it is suspended from use, at this time the ventilation therapy apparatus may automatically switch to the "hot standby" mode: the fan runs at a lower fourth preset rotating speed, and outputs a very small airflow, the oxygen proportional valve is closed, and the humidifier works normally to maintain the temperature which may provide normal temperature and humidity output at any time; after detecting that the patient wears the patient interface, the ventilation therapy apparatus outputs airflow according to the normal working mode, after the patient removes the patient interface, the ventilation therapy apparatus may return to the "hot standby" mode again.

Optionally, the control module is configured for detecting output parameters of the main body; the control module is further configured for monitoring the patient's respiratory flow value; when it is determined that the main body is in a flow priority state, the control module is configured for adjusting the output parameters of the main body to be larger than the patient's respiratory flow value; and when it is determined that the main body is in a pressure priority state, the control module is configured for adjusting the output parameters of the main body to be a positive pressure value.

In the embodiment of the present disclosure, the output of the ventilation therapy apparatus may be in one of the following two modes:

the first is the flow priority mode: that is the patient's respiratory flow value is calculated at any time, to ensure that the output flow value of the ventilation therapy apparatus is larger than the patient's respiratory flow value, so as to ensure that all the gas inhaled by the patient is provided by the ventilation therapy apparatus, and will not inhale air through the gap between the patient interface and the nasal cavity.

the second is the pressure priority mode: in any respiratory state of the patient, the apparatus must output sufficient airflow, to ensure that the disposed positive pressure value is maintained in the patient's nasal cavity. The positive pressure value is larger than the atmospheric pressure value, because in the embodiment of the present disclosure, if the ventilation treatment apparatus uses an open air path, it must be ensured that the positive pressure is maintained in the nasal cavity, to ensure that the human body does not directly inhale external air.

Optionally, the respiratory pipe and the main body are connected through a gas path and a circuit, and the circuit and the gas path are on and off simultaneously.

In the embodiment of the present disclosure, the respiratory pipe and the main body are connected through the gas path, which may output the gas provided by the main body to the patient. In addition, the respiratory pipe and the main body are connected through the circuit, and the electrical device in the respiratory pipe may also be electrically connected to the main body, to realize the corresponding functions of the electrical device. For example, the electrical device may include a humidifier, heating elements and temperature sensors, these devices need to be powered by the main body, need to receive control signals transmitted by the main body, and at the same time need to transmit corresponding status signals to the main body.

In summary, the ventilation therapy apparatus according to the embodiment of the present disclosure, includes: the main body, the respiratory pipe, the patient interface, the oxygen supply module, the oxygen proportional valve and the control module; the main body is configured for outputting gas with a preset pressure and a preset flow, and the main body comprises an output end; the respiratory pipe comprises a first end and a second end which communicates with each other, and the first end of the respiratory pipe communicates with the output end; the second end of the respiratory pipe is connected to the patient interface, the patient interface is configured for being worn on a patient's nasal cavity, when the patient interface is worn on the patient's nasal cavity, an air outlet gap is disposed between the patient interface and the patient's nasal cavity; the oxygen supply module is connected to the main body through the oxygen proportional valve, the control module is configured for detecting output parameters of the main body, and when it is determined that the main body is in a preset state, the control module controls the oxygen proportional valve to open at a corresponding preset opening degree according to the output parameters, and controls the fan of the main body to run at a corresponding preset rotating speed. The present disclosure uses the feedback of the output parameters output by the main body to determine the patient's expiratory and inspiratory flow, and adjusts the opening degree of the oxygen proportional valve and the rotating speed of the fan of the main body, to deliver the airflow corresponding to the patient's respiratory flow or pressure to the patient, so that the patient may receive a constant concentration oxygen more comfortably, and improve the user's respiratory experience. The embodiment of the present disclosure controls the oxygen proportional valve to open at different opening degrees by the control module according to different breathing states, so that the corresponding flow oxygen may be continuously provided with the patient's breathing, which ensures the open air path of the respiratory pipe to realize continuous positive pressure output, and the patient will not inhale air from the external environment, and provides the patient with a constant concentration of oxygen, which is convenient for the doctor to formulate the treatment plan and confirm the treatment effect.

Referring to Fig. 7, it is shown a flow chart of the method for controlling the ventilation therapy apparatus according the present disclosure, comprises:

Step 701, detecting output parameters of a main body.

In the embodiment of the present disclosure, the main body may output an output parameter to the patient through the respiratory pipe, and the feedback of the output parameter may be configured to calculate the airflow pressure at the patient interface.

Step 702, when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air.

In this step, the feedback obtained by the output parameters may calculate the airflow pressure at the patient interface, and according to the change of the airflow pressure at the patient interface, to identify the patient's expiratory and inspiratory flow, which realizes the monitoring of the flow change during the patient's breathing, so that the main body realizes real-time adjustment of output flow and pressure, that is keeping the output flow slightly larger than the patient's inhalation flow or keeping the pressure in the patient's nasal cavity is always positive pressure. At the same time, during the oxygen supply process, by adjusting the opening degree of the oxygen proportional valve, realizing the real-time adjustment of the oxygen flow rate provided by the oxygen supply module, which may keep the output oxygen concentration to be a fixed value. Therefore, in the embodiments of the present disclosure, the patient may receive a stable concentration of oxygen more comfortably by delivering an airflow slightly higher than the patient's breathing flow or pressure to the patient.

Step 703, mixing the air inhaled by the main body with the oxygen supplied by the oxygen supply module, and obtaining a gas mixture.

In this step, the main body mixes the inhaled air with the oxygen supplied by the oxygen supply module, and obtains the gas mixture, in the gas mixture, the oxygen concentration is constant.

Step 704, outputting the gas mixture through a respiratory pipe.

In summary, the method for controlling the ventilation therapy apparatus according to the embodiment of the present disclosure, includes: detecting output parameters of a main body; when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air; mixing the air inhaled by the main body with the oxygen supplied by the oxygen supply module, and obtaining a gas mixture; and outputting the gas mixture through a respiratory pipe. The present disclosure uses the feedback of the output parameters of the main body to determine the patient's expiratory and inspiratory flow, and adjusts the opening degree of the oxygen proportional valve and the rotating speed of the fan of the main body to deliver the airflow corresponding to the patient's respiratory flow or pressure to the patient, so that the patient may receive a constant concentration oxygen more comfortably, and improve the user's respiratory experience.

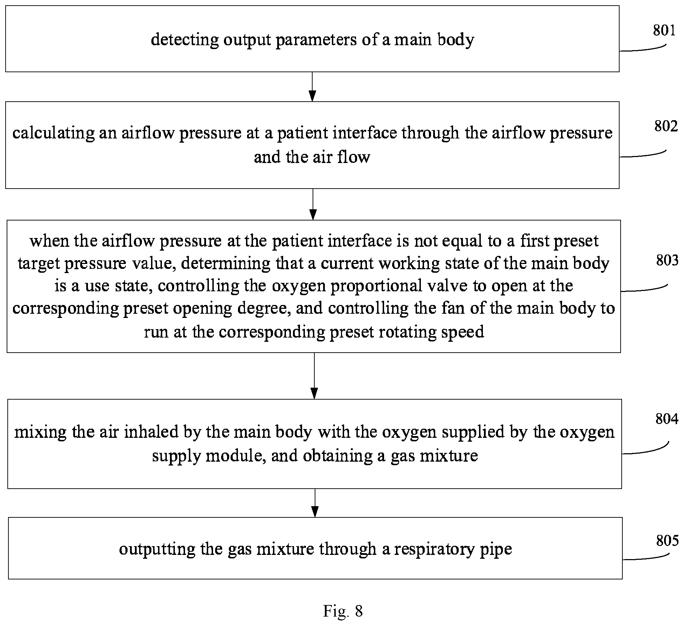

Referring to Fig. 8, it is shown a specific flow chart of the method for controlling the ventilation therapy apparatus according to the present disclosure, comprises:

Step 801, detecting output parameters of a main body.

For details of this step, reference may be made to the above description of the step 701, which is not repeated here.

Step 802, calculating an airflow pressure at a patient interface through the airflow pressure and the air flow.

In this step, for the specific implementation of calculating the airflow pressure at a patient interface through the airflow pressure and the air flow, reference may be made to the relevant description in the embodiment of the ventilation therapy apparatus according to the present disclosure, which will not be repeated here.

Step 803, when the airflow pressure at the patient interface is not equal to a first preset target pressure value, determining that a current working state of the main body is a use state, controlling the oxygen proportional valve to open at the corresponding preset opening degree, and controlling the fan of the main body to run at the corresponding preset rotating speed.

Optionally, step 803 may also include sub-step:

sub-step 8031, if the airflow pressure at the patient interface is less than the first preset target pressure value, determining that the current working state of the main body is an inspiratory state, controlling the oxygen proportional valve to open at a corresponding second opening degree, and controlling the fan of the main body to run at a corresponding second preset rotating speed.

In this step, before the patient uses the ventilation therapy apparatus, the first preset target pressure value $P_t$ will be preset according to their own conditions, and the first preset target pressure value $P_t$ is the pressure value in an ideal state free from the interference of the pressure drop. When the patient uses the ventilation therapy apparatus, due to the interference of the pressure drop, which will cause the actual pressure $P_2$ at the patient interface is different from the first preset target pressure value Pt, and the influence of this pressure drop on the actual pressure $P_2$ at the patient interface may be determined by the comparison result between $P_2$ and $P_t$. Generally, if the airflow pressure $P_2$ at the patient interface is less than the first preset target pressure value $P_t$, it is determined that the current working state of the main body is the inhalation state.

If it is determined that the current working state of the main body is the inhalation state, the control module controls the oxygen proportional valve to open at the corresponding second opening degree, and controls the fan of the main body to run at the corresponding second preset rotating speed, its meaning is that when the patient is inhaling, the main body mixes more inhaled oxygen provided by the oxygen supply module with more air inhaled by the fan, so that the output flow of the ventilation therapy apparatus is slightly larger than the patient's inhalation volume. At the same time, the oxygen concentration is kept constant for auxiliary inhalation.

Sub-step 8032, if the airflow pressure at the patient interface is larger than the first preset target pressure value, determining that the current working state of the main body is an exhalation state, controlling the oxygen proportional valve to open at a corresponding third opening degree, and controlling the fan of the main body to run at a corresponding third preset rotating speed.

If the airflow pressure $P_2$ at the patient interface is larger than the first preset target pressure value $P_t$, it is determined that the current working state of the main body is the exhalation state.

When it is determined the patient is exhaling, the main body mixes less inhaled oxygen provided by the oxygen supply module with less air inhaled by the fan, so that the ventilation therapy apparatus outputs a small flow rate, while maintaining a constant oxygen concentration, so as to prevent the patient's exhaled gas from flowing back to the ventilation therapy apparatus.

Sub-step 8033, when the airflow pressure at the patient interface is equal to a second preset target pressure value and is maintained for a preset time, determining that the current working state of the main body is a non-use state, controlling the oxygen proportional valve to open at a corresponding first opening degree, and controlling the fan of the main body to run at a corresponding first preset rotating speed.

In the embodiment of the present disclosure, when the airflow pressure at the patient interface is equal to the second preset target pressure value, and it is maintained for the preset time, it shows that at this moment the patient does not use the ventilation therapy apparatus, it is the standby mode, the patient interface exposes to the air, the control module controls the oxygen proportional valve to open at the corresponding first opening degree, and meanwhile controls the fan of the main body to run at the corresponding first preset rotating speed, to continually output a small pressure and flow, which may ensure the temperature and the humidity inside the respiratory pipe to be constant.

Optionally, the second opening degree is larger than the first opening degree, the first opening degree is larger than or equal to the third opening degree; the second preset rotating speed is larger than the first preset rotating speed, the first preset rotating speed is larger than or equal to the third preset rotating speed. Wherein, the first opening degree may be equal to the third opening degree; the first preset rotating speed may also be equal to the third preset rotating speed. As long as it is ensured that both the first opening degree and the third opening degree are less than the second opening degree; the first preset rotating speed and the third preset rotating speed are both less than the second preset rotating speed.

Optionally, step 803 may also include sub-step:

sub-step 8034, when the airflow pressure at the patient interface is equal to the second preset target pressure value, and is maintained for the preset time, determining that the current working state of the main body is the standby state, controlling the oxygen proportional valve to close, and controlling a humidifier to start, and controlling the fan of the main body to run at a corresponding fourth preset rotating speed; wherein the fourth preset rotating speed is less than the first preset rotating speed.

In the embodiment of the present disclosure, when the airflow pressure at the interface of the patient is equal to the second preset target pressure value, and it is maintained for the preset time, it is determined that the current working state of the main body is the standby state. At this time, it is specifically the state that the ventilation therapy apparatus is not connected to the patient after starting, or the patient removes the patient interface and it is suspended from use, at this time the ventilation therapy apparatus may automatically switch to the "hot standby" mode: the fan runs at a lower fourth preset rotating speed, and outputs a very small airflow, the oxygen proportional valve is closed, and the humidifier works normally to maintain the temperature which may provide normal temperature and humidity output at any time; after detecting that the patient wears the patient interface, the ventilation therapy apparatus outputs airflow according to the normal working mode, after the patient removes the patient interface, the ventilation therapy apparatus may return to the "hot standby" mode again.

Optionally, step 803 may also include sub-step:

sub-step 8035, monitoring a respiratory flow value of the patient.

Sub-step 8036, when it is determined that the main body is in a flow priority state, adjusting the output parameters of the main body to be larger than the respiratory flow value of the patient.

This step provides a flow priority mode: that is the patient's respiratory flow value is calculated at any time, to ensure that the output flow value of the ventilation therapy apparatus is larger than the patient's respiratory flow value, so as to ensure that all the gas inhaled by the patient is provided by the ventilation therapy apparatus, and will not inhale air through the gap between the patient interface and the nasal cavity.

Sub-step 8037, when it is determined that the main body is in a pressure priority state, adjusting the output parameters of the main body to be a positive pressure value.

This step provides a pressure priority mode: in any respiratory state of the patient, the apparatus must output sufficient airflow, to ensure that the disposed positive pressure value is maintained in the patient's nasal cavity. The positive pressure value is larger than the atmospheric pressure value, because in the embodiment of the present disclosure, if the ventilation treatment apparatus uses an open air path, it must be ensured that the positive pressure is maintained in the nasal cavity, to ensure that the human body does not directly inhale external air.

In the embodiment of the present disclosure, when the airflow pressure at the interface of the patient is equal to the second preset target pressure value, and it is maintained for the preset time, it is determined that the current working state of the main body is the standby state. At this time, it is specifically the state that the ventilation therapy apparatus is not connected to the patient after starting, or the patient removes the patient interface and it is suspended from use, at this time the ventilation therapy apparatus may automatically switch to the "hot standby" mode: the fan runs at a lower fourth preset rotating speed, and outputs a very small airflow, the oxygen proportional valve is closed, and the humidifier works normally to maintain the temperature which may provide normal temperature and humidity output at any time; after detecting that the patient wears the patient interface, the ventilation therapy apparatus outputs airflow according to the normal working mode, after the patient removes the patient interface, the ventilation therapy apparatus may return to the "hot standby" mode again.

Step 804, mixing the air inhaled by the main body with the oxygen supplied by the oxygen supply module, and obtaining a gas mixture.

For details of this step, reference may be made to the above description of the step 703, which is not repeated here.

Step 805, outputting the gas mixture through a respiratory pipe.

For details of this step, reference may be made to the above description of the step 704, which is not repeated here.

In summary, the method for controlling the ventilation therapy apparatus according to the embodiment of the present disclosure, includes: detecting output parameters of a main body; when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air; mixing the air inhaled by the main body with the oxygen supplied by the oxygen supply module, and obtaining a gas mixture; and outputting the gas mixture through a respiratory pipe. The present disclosure uses the feedback of the output parameters of the main body to determine the patient's expiratory and inspiratory flow, and adjusts the opening degree of the oxygen proportional valve and the rotating speed of the fan of the main body to deliver the airflow corresponding to the patient's respiratory flow or pressure to the patient, so that the patient may receive a constant concentration oxygen more comfortably, and improve the user's respiratory experience. The embodiment of the present disclosure controls the oxygen proportional valve to open at different opening degrees by the control module according to different breathing states, so that the corresponding flow oxygen may be continuously provided with the patient's breathing, which ensures the open air path of the respiratory pipe to realize continuous positive pressure output, and the patient will not inhale air from the external environment, and provides the patient with a constant concentration of oxygen, which is convenient for the doctor to formulate the treatment plan and confirm the treatment effect.

Each component embodiment of the present application may be implemented by hardware, or by software modules that are operated on one or more processors, or by a combination thereof. A person skilled in the art should understand that some or all of the functions of some or all of the components of the calculating and processing device according to the embodiments of the present application may be implemented by using a microprocessor or a digital signal processor (DSP) in practice. The present application may also be implemented as apparatus or device programs (for example, computer programs and computer program products) for implementing part of or the whole of the method described herein. Such programs for implementing the present application may be stored in a computer-readable medium, or may be in the form of one or more signals. Such signals may be downloaded from an Internet website, or provided on a carrier signal, or provided in any other forms.

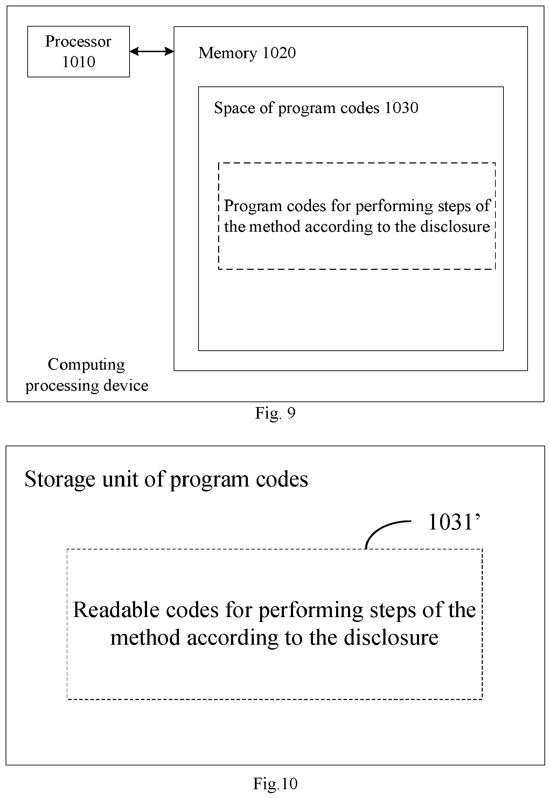

For example, Fig. 9 shows a calculating and processing device that can implement the method according to the present application. The calculating and processing device traditionally comprises a processor 1010 and a computer program product or computer-readable medium in the form of a memory 1020. The memory 1020 may be electronic memories such as flash memory, EEPROM (Electrically Erasable Programmable Read Only Memory), EPROM, hard disk or ROM. The memory 1020 has the storage space 1030 of the program code 1031 for implementing any steps of the above method. For example, the storage space 1031 for program code may contain program codes 1031 for individually implementing each of the steps of the above method. Those program codes may be read from one or more computer program products or be written into the one or more computer program products. Those computer program products include program code carriers such as hard disk, compact disk (CD), memory card or floppy disk as shown in Fig. 10. Such computer program products are usually portable or fixed storage units. The storage unit may have storage segments or storage spaces with similar arrangement to the memory 1020 of the calculating and processing device in Fig. 9. The program codes may for example be compressed in a suitable form. Generally, the storage unit contains a computer-readable code 1031', which can be read by a processor like 1010. When those codes are executed by the calculating and processing device, the codes cause the calculating and processing device to implement each of the steps of the method described above.

In the present application, a computer-readable storage medium includes any mechanism for storing or transmitting information in a form readable by a computer (for example, a computer). For example, the computer-readable storage medium includes a read-only memory (ROM), a random access memory (RAM), a magnetic-disk storage medium, an optical storage medium, a flash storage medium, a propagation signal in an electric, optical, acoustic or other form (for example, a carrier wave, an infrared signal and a digital signal) and so on.

## Claims

1. A ventilation therapy apparatus, comprising: a main body (10), a respiratory pipe (20), a patient interface (203), an oxygen supply module (30), an oxygen proportional valve (40) and a control module (50); wherein,

the main body (10) is configured for outputting gas with a preset pressure and a preset flow, and the main body (10) comprises an output end;
the respiratory pipe (20) comprises a first end (201) and a second end (202) which communicates with each other, and the first end (201) of the respiratory pipe (20) communicates with the output end;
the second end (202) of the respiratory pipe (20) is connected to the patient interface (203), the patient interface (203) is configured for being worn on a patient's nasal cavity, and when the patient interface (203) is worn on the patient's nasal cavity, an air outlet gap is disposed between the patient interface (203) and the patient's nasal cavity;

the oxygen supply module (30) is connected to the main body (10) through the oxygen proportional valve (40), and a fan (101) is disposed in the main body (10);

the main body (10) is configured to mix an inhaled air with the oxygen provided by the oxygen supply module (30), and output a gas mixture through the respiratory pipe (20); the control module (50) is connected to the main body (10) and the oxygen proportional valve (40), respectively; and

the control module (50) is configured for detecting output parameters of the main body (10), the output parameters comprising an airflow pressure and an air flow at the output end of the main body (10),

when it is determined that the main body (10) is in a preset state, the control module (50), according to the output parameters, is configured to:

> control the oxygen proportional valve (40) to open at a corresponding preset opening degree, and
> control the fan (101) of the main body (10) to run at a corresponding preset rotating speed.

2. The ventilation therapy apparatus according to claim 1, wherein,

> the control module (50) is configured for calculating an airflow pressure at the patient interface through the airflow pressure and the air flow; and
> when the calculated airflow pressure at the patient interface (203) is not equal to a first preset target pressure value, it is determined that a current working state of the main body (10) is a use state, the control module (50) is configured for controlling the oxygen proportional valve (40) to open at the corresponding preset opening degree, and controlling the fan (101) of the main body (10) to run at the corresponding preset rotating speed.

3. The ventilation therapy apparatus according to claim 2, wherein the operation that when

> the calculated airflow pressure at the patient interface (203) is not equal to the first preset target pressure value, it is determined that a current working state of the main body (10) is a use state, the control module (50) is configured for controlling the oxygen proportional valve (40) to open at the corresponding preset opening degree, and controlling the fan (101) of the main body (10) to run at the corresponding preset rotating speed, comprises:
> if the calculated airflow pressure at the patient interface (203) is less than the first preset target pressure value, it is determined that the current working state of the main body (10) is an inspiratory state, the control module (50) is further configured for controlling the oxygen proportional valve (40) to open at a corresponding second opening degree, and controlling the fan (101) of the main body (10) to run at a corresponding second preset rotating speed; and
> if the calculated airflow pressure at the patient interface (203) is larger than the first preset target pressure value, it is determined that the current working state of the main body (10) is an exhalation state, the control module (50) is further configured for controlling the oxygen proportional valve (40) to open at a corresponding third opening degree, and controlling the fan (101) of the main body (10) to run at a corresponding third preset rotating speed.

4. The ventilation therapy apparatus according to claim 2 or 3, wherein when the calculated airflow pressure at the patient interface (203) is equal to a second preset target pressure value and is maintained for a preset time, it is determined that the current working state of the main body (10) is a non-use state, the control module (50) is further configured for controlling the oxygen proportional valve (40) to open at a corresponding first opening degree, and controlling the fan (101) of the main body (10) to run at a corresponding first preset rotating speed;

wherein the second preset target pressure value is less than the first preset target pressure value.

5. The ventilation therapy apparatus according to claim 4, wherein the second opening degree is larger than the first opening degree, the first opening degree is larger than or equal to the third opening degree; the second preset rotating speed is larger than the first preset rotating speed, and the first preset rotating speed is larger than or equal to the third preset rotating speed.

6. The ventilation therapy apparatus according to any one of claims 1 to 5, wherein the ventilation therapy apparatus further comprises:

a humidifier, configured for heating and humidifying the output gas.

7. The ventilation therapy apparatus according to claim 6, wherein when the calculated airflow pressure at the patient interface (203) is equal to the second preset target pressure value and is maintained for a preset time, it is determined that the current working state of the main body (10) is a standby state, the control module (50) is further configured for controlling the oxygen proportional valve (40) to close, and controlling the humidifier to start, and controlling the fan (101) of the main body (10) to run at a corresponding fourth preset rotating speed;
wherein the fourth preset rotating speed is less than the first preset rotating speed.

8. The ventilation therapy apparatus according to any one of claims 1 to 7, wherein the operation that the control module (50) is configured for detecting output parameters of the main body (10), when it is determined that the main body (10) is in a preset state, the control module (50) controls the oxygen proportional valve (40) to open at a corresponding preset opening degree according to the output parameters, and controls the fan (101) of the main body (10) to run at a corresponding preset rotating speed, comprises:

the control module (50) is configured for detecting the output parameters of the main body (10); and the control module (50) is further configured for monitoring a respiratory flow value of the patient;
when it is determined that the main body (10) is in a flow priority state, the control module (50) is configured for adjusting the output parameters of the main body (10) to be larger than the respiratory flow value of the patient; and
when it is determined that the main body (10) is in a pressure priority state, the control module (50) is configured for adjusting the output parameters of the main body (10) to be a positive pressure value.

9. The ventilation therapy apparatus according to any one of claims 1 to 8, wherein the respiratory pipe (20) and the main body (10) are connected through a gas path and a circuit, and the circuit and the gas path are on and off simultaneously.

10. A computer program comprising a computer readable code, when the computer readable code is run on a computing processing device of a control module (50) of a ventilation therapy apparatus according to any one of claims 1 to 9, causing the computing processing device to execute a method for controlling the ventilation therapy apparatus, wherein the method comprises:

detecting output parameters of a main body (10), the output parameters comprising an airflow pressure and an air flow at the output end of the main body (10);
when it is determined that the main body (10) is in a preset state, according to the output parameters,

controlling an oxygen proportional valve (40) connected to the main body (10) to open at a corresponding preset opening degree, to allow an oxygen supply module (30) to supply oxygen for the main body (10) through the oxygen proportional valve (40), and
controlling a fan (101) of the main body (10) to run at a corresponding preset rotating speed, to allow the main body (10) to inhale air;
mixing the air inhaled by the main body (10) with the oxygen supplied by the oxygen supply module (30), and obtaining a gas mixture; and
outputting the gas mixture through a respiratory pipe (20).

11. The computer program according to claim 10, wherein,

the step of when it is determined that the main body (10) is in a preset state, controlling an oxygen proportional valve (40) connected to the main body (10) to open at a corresponding preset opening degree according to the output parameters, to allow an oxygen supply module (30) to supply oxygen for the main body (10) through the oxygen proportional valve (40), and controlling a fan (101) of the main body (10) to run at a corresponding preset rotating speed, to allow the main body (10) to inhale air comprises: calculating an airflow pressure at a patient interface (203) through the airflow pressure and the air flow; and
when the calculated airflow pressure at the patient interface (203) is not equal to a first preset target pressure value, determining that a current working state of the main body (10) is a use state, controlling the oxygen proportional valve (40) to open at the corresponding preset opening degree, and controlling the fan (101) of the main body (10) to run at the corresponding preset rotating speed.

12. The computer program according to claim 11, wherein the step of when the calculated airflow pressure at the patient interface (203) is not equal to a first preset target pressure value, determining that a current working state of the main body (10) is a use state, controlling the oxygen proportional valve (40) to open at the corresponding preset opening degree, and controlling the fan (101) of the main body (10) to run at the corresponding preset rotating speed, comprises:

if the calculated airflow pressure at the patient interface (203) is less than the first preset target pressure value, determining that the current working state of the main body (10) is an inspiratory state, controlling the oxygen proportional valve (40) to open at a corresponding second opening degree, and controlling the fan (101) of the main body (10) to run at a corresponding second preset rotating speed; and

if the claculated airflow pressure at the patient interface (203) is larger than the first preset target pressure value, determining that the current working state of the main body (10) is an exhalation state, controlling the oxygen proportional valve (40) to open at a corresponding third opening degree, and controlling the fan (101) of the main body (10) to run at a corresponding third preset rotating speed.

**13.** The computer program according to claim 11 or 12, wherein the method further comprises:

when the calculated airflow pressure at the patient interface (203) is equal to a second preset target pressure value, and is maintained for a preset time, determining that the current working state of the main body (10) is a non-use state, controlling the oxygen proportional valve (40) to open at a corresponding first opening degree, and controlling the fan (101) of the main body (10) to run at a corresponding first preset rotating speed; wherein the second preset target pressure value is less than the first preset target pressure value.

**14.** The computer program according to claim 13, wherein the method further comprises:

when the calculated airflow pressure at the patient interface (203) is equal to the second preset target pressure value, and is maintained for the preset time, determining that the current working state of the main body (10) is a standby state, controlling the oxygen proportional valve (40) to close, and controlling a humidifierto start, and controlling the fan (101) of the main body (10) to run at a corresponding fourth preset rotating speed; and wherein the fourth preset rotating speed is less than the first preset rotating speed.

**Patentansprüche**

**1.** Beatmungstherapie-Vorrichtung, die umfasst: einen Hauptkörper (10), eine Beatmungsleitung (20), eine Patienten-Schnittstelle (203), ein Sauerstoffzufuhr-Modul (30), ein Sauerstoff-Proportionalventil (40) sowie ein Steuerungs-Modul (50); wobei

der Hauptkörper (10) so ausgeführt ist, dass er Gas mit einem voreingestellten Druck und einem voreingestellten Strom ausgibt, und der Hauptkörper (10) ein Ausgangsende umfasst;

die Beatmungsleitung (20) ein erstes Ende (201) und ein zweites Ende (202) umfasst, die miteinander in Verbindung stehen, und das erste Ende (201) der Beatmungsleitung (20) mit dem Ausgangsende in Verbindung steht;

das zweite Ende (202) der Beatmungsleitung (20) mit der Patienten-Schnittstelle (203) verbunden ist, wobei die Patienten-Schnittstelle (203) so ausgeführt ist, dass sie auf einer Nasenhöhle des Patienten getragen wird, und wenn die Patienten-Schnittstelle (203) auf der Nasenhöhle des Patienten getragen wird, ein Luftauslassspalt zwischen der Patienten-Schnittstelle (203) und der Nasenhöhle des Patienten angeordnet ist;

das Sauerstoffzufuhr-Modul (30) über das Sauerstoff-Proportionalventil (40) mit dem Hauptkörper (10) verbunden ist, und ein Gebläse (101) in dem Hauptkörper (10) angeordnet ist;

der Hauptkörper (10) so ausgeführt ist, dass er eingeatmete Luft mit dem von dem Sauerstoffzufuhr-Modul (30) bereitgestellten Sauerstoff mischt und ein Gasgemisch über die Beatmungsleitung (20) ausgibt;

das Steuerungs-Modul (50) mit dem Hauptkörper (10) bzw. dem Sauerstoff-Proportionalventil (40) verbunden ist; und

das Steuerungs-Modul (50) so ausgeführt ist, dass es Ausgangsparameter des Hauptkörpers (10) erfasst, wobei die Ausgangsparameter einen Luftstromdruck sowie einen Luftstrom an dem Ausgangsende des Hauptkörpers (10) umfassen,

das Steuerungs-Modul (50) so ausgeführt ist, dass es, wenn festgestellt wird, dass sich der Hauptkörper in einem voreingestellten Zustand befindet, konfiguriert ist zum:

Steuern des Sauerstoff-Proportionalventils (40) so, dass es sich mit einem entsprechenden voreingestellten Öffnungsgrad öffnet, sowie
Steuern des Gebläses (101) des Hauptkörpers (10) so, dass es mit einer entsprechenden voreingestellten Drehzahl läuft.

2. Beatmungstherapie-Vorrichtung nach Anspruch 1, wobei
das Steuerungs-Modul (50) so ausgeführt ist, dass es einen Luftstromdruck an der Patienten-Schnittstelle anhand des Luftstromdrucks sowie des Luftstroms berechnet; und das Steuerungs-Modul (50) so ausgeführt ist, dass es, wenn der berechnete Luftstromdruck an der Patienten-Schnittstelle (203) einem ersten voreingestellten Druck-Zielwert nicht gleich ist und festgestellt wird, dass ein aktueller Betriebszustand des Hauptkörpers (10) ein Einsatzzustand ist, das Sauerstoff-Proportionalventil (40) so steuert, dass es sich mit dem entsprechenden voreingestellten Öffnungsgrad öffnet, und das Gebläse (101) des Hauptkörpers (10) so steuert, dass es mit der entsprechenden voreingestellten Drehzahl läuft.

3. Beatmungstherapie-Vorrichtung nach Anspruch 2, wobei der Betrieb, bei dem das Steuerungs-Modul (50) so ausgeführt ist, dass es, wenn der berechnete Luftstromdruck an der Patienten-Schnittstelle (203) dem ersten voreingestellten Druck-Zielwert nicht gleich ist und festgestellt wird, dass ein aktueller Betriebszustand des Hauptkörpers (10) ein Einsatzzustand ist, das Sauerstoff-Proportionalventil (40) so steuert, dass es sich mit dem entsprechenden voreingestellten Öffnungsgrad öffnet, und das Gebläse (101) des Hauptkörpers (10) so steuert, dass es mit der entsprechenden voreingestellten Drehzahl läuft, umfasst, dass:

das Steuerungs-Modul (50) des Weiteren so ausgeführt ist, dass es, wenn der berechnete Luftstromdruck an der Patienten-Schnittstelle (203) niedriger ist als der erste voreingestellte Druck-Zielwert und festgestellt wird, dass der aktuelle Betriebszustand des Hauptkörpers (10) ein Einatmungs-Zustand ist, das Sauerstoff-Proportionalventil (40) so steuert, dass es sich mit einem entsprechenden zweiten Öffnungsgrad öffnet, und das Gebläse (101) des Hauptkörpers (10) so steuert, dass es mit einer entsprechenden zweiten voreingestellten Drehzahl läuft; und
das Steuerungs-Modul (50) des Weiteren so ausgeführt ist, dass es, wenn der berechnete Luftstromdruck an der Patienten-Schnittstelle (203) über dem ersten voreingestellten Druck-Zielwert liegt und festgestellt wird, dass der aktuelle Betriebszustand des Hauptkörpers (10) ein Ausatmungs-Zustand ist, das Sauerstoff-Proportionalventil (40) so steuert, dass es sich mit einem entsprechenden dritten Öffnungsgrad öffnet, und das Gebläse (101) des Hauptkörpers (10) so steuert, dass es mit einer entsprechenden dritten voreingestellten Drehzahl läuft.

4. Beatmungstherapie-Vorrichtung nach Anspruch 2 oder 3, wobei das Steuerungs-Modul des Weiteren so ausgeführt ist, dass es, wenn der berechnete Luftstromdruck an der Patienten-Schnittstelle (203) einem zweiten voreingestellten Druck-Zielwert gleich ist und über eine voreingestellte Zeit aufrechterhalten wird und festgestellt wird, dass der aktuelle Betriebszustand des Hauptkörpers (10) ein Ruhezustand ist, das Sauerstoff-Proportionalventil (40) so steuert, dass es sich mit einem entsprechenden ersten Öffnungsgrad öffnet, und das Gebläse (101) des Hauptkörpers (10) so steuert, dass es mit einer entsprechenden ersten voreingestellten Drehzahl läuft;
wobei der zweite voreingestellte Druck-Zielwert kleiner ist als der erste voreingestellte Druck-Zielwert.

5. Beatmungstherapie-Vorrichtung nach Anspruch 4, wobei der zweite Öffnungsgrad größer ist als der erste Öffnungsgrad, der erste Öffnungsgrad größer ist als oder genauso groß wie der dritte Öffnungsgrad, die zweite voreingestellte Drehzahl höher ist als die erste voreingestellte Drehzahl, und die erste voreingestellte Drehzahl höher ist als oder genauso hoch wie die dritte voreingestellte Drehzahl.

6. Beatmungstherapie-Vorrichtung nach einem der Ansprüche 1 bis 5, wobei die Beatmungstherapie-Vorrichtung des Weiteren umfasst:
einen Befeuchter, der zum Erwärmen und Befeuchten des Ausgangsgases ausgeführt ist.

7. Beatmungstherapie-Vorrichtung nach Anspruch 6, wobei das Steuerungs-Modul (50) des Weiteren so ausgeführt ist, dass es wenn der berechnete Luftstromdruck an der Patienten-Schnittstelle (203) dem zweiten voreingestellten Druck-Zielwert gleich ist und über eine voreingestellte Zeit aufrechterhalten wird und festgestellt wird, dass der aktuelle Betriebszustand des Hauptkörpers (10) ein Bereitschaftszustand ist, das Sauerstoff-Proportionalventil (40) so steuert, dass es sich schließt, und den Befeuchter so steuert, dass er startet, und das Gebläse (101) des Hauptkörpers (10) so steuert, dass es mit einer entsprechenden vierten voreingestellten Drehzahl läuft;
wobei die vierte voreingestellte Drehzahl niedriger ist als die erste voreingestellte Drehzahl.

8. Beatmungstherapie-Vorrichtung nach einem der Ansprüche 1 bis 7, wobei der Betrieb, bei dem das Steuerungs-Modul (50) so ausgeführt ist, dass es Ausgangsparameter des Hauptkörpers (10) erfasst und das Steuerungs-Modul (50), wenn festgestellt wird, dass sich der Hauptkörper (10) in einem voreingestellten Zustand befindet, das Sauerstoff-Proportionalventil (40) entsprechend den Ausgangsparametern so steuert, dass es sich mit einem entsprechenden voreingestellten Öffnungsgrad öffnet, und das Gebläse (101) des Hauptkörpers (10) so steuert, dass er mit einer entsprechenden voreingestellten Drehzahl läuft, umfasst, dass:

das Steuerungs-Modul (50) so ausgeführt ist, dass es die Ausgangsparameter des Hauptkörpers (10) erfasst; und das Steuerungs-Modul (50) des Weiteren so ausgeführt ist, dass es einen Atemstrom-Wert des Patienten überwacht;

das Steuerungs-Modul (50) so ausgeführt ist, dass es, wenn festgestellt wird, dass sich der Hauptkörper in einem Strom-Prioritätszustand befindet, die Ausgangsparameter des Hauptkörpers (10) so einstellt, dass sie größer sind als der Atemstrom-Wert des Patienten; und

das Steuerungs-Modul (50) so ausgeführt ist, dass es, wenn festgestellt wird, dass sich der Hauptkörper in einem Druck-Prioritätszustand befindet, die Ausgangsparameter des Hauptkörpers (10) so einstellt, dass sie ein positiver Druckwert sind.

9. Beatmungstherapie-Vorrichtung nach einem der Ansprüche 1 bis 8, wobei die Beatmungsleitung (20) und der Hauptkörper (10) über einen Gasweg sowie einen Kreis verbunden sind und der Kreis sowie der Gasweg gleichzeitig geöffnet und geschlossen werden.

10. Computerprogramm, das einen computerlesbaren Code umfasst, wobei, wenn der computerlesbare Code auf einer Computer-Verarbeitungsvorrichtung eines Steuerungs-Moduls (50) einer Beatmungstherapie-Vorrichtung nach einem der Ansprüche 1 bis 9 ausgeführt wird, er die Computer-Verarbeitungsvorrichtung veranlasst, ein Verfahren zum Steuern der Beatmungstherapie-Vorrichtung auszuführen, wobei das Verfahren umfasst:

Erfassen von Ausgangsparametern eines Hauptkörpers (10), wobei die Ausgangsparameter einen Luftstromdruck sowie einen Luftstrom an dem Ausgangsende des Hauptkörpers (10) umfassen;

wenn entsprechend den Ausgangsparametern festgestellt wird, dass sich der Hauptkörper (10) in einem voreingestellten Zustand befindet,

Steuern eines mit dem Hauptkörper (10) verbundenen Sauerstoff-Proportionalventils (40) so, dass es sich mit einem entsprechenden voreingestellten Öffnungsgrad öffnet, um es einem Sauerstoffzufuhr-Modul (30) zu ermöglichen, Sauerstoff für den Hauptkörper (10) über das Sauerstoff-Proportionalventil (40) zuzuführen, sowie

Steuern eines Gebläses (101) des Hauptkörpers (10) so, dass es mit einer entsprechenden voreingestellten Drehzahl läuft, um es dem Hauptkörper (10) zu ermöglichen, Luft zu inhalieren;

Mischen der durch den Hauptkörper (10) inhalierten Luft mit dem durch das Sauerstoffzufuhr-Modul (30) zugeführten Sauerstoff sowie Erzeugen eines Gasgemischs; und

Ausgeben des Gasgemischs über eine Beatmungsleitung (20).

11. Computerprogramm nach Anspruch 10, wobei
der Schritt, in dem festgestellt wird, dass sich der Hauptkörper (10) in einem voreingestellten Zustand befindet, ein mit dem Hauptkörper (10) verbundenes Sauerstoff-Proportionalventil (40) so gesteuert wird, dass es sich mit einem entsprechenden voreingestellten Öffnungsgrad gemäß den Ausgangsparametern öffnet, um es einem Sauerstoffzufuhr-Modul (30) zu ermöglichen, Sauerstoff für den Hauptkörper (10) über das Sauerstoff-Proportionalventil (40) zuzuführen, und ein Gebläse (101) des Hauptkörpers (10), so gesteuert wird, dass es mit einer entsprechenden voreingestellten Drehzahl läuft, um es dem Hauptkörper (10) zu ermöglichen, Luft zu inhalieren, umfasst:

Berechnen eines Luftstromdrucks an einer Patienten-Schnittstelle (203) anhand des Luftstromdrucks sowie des Luftstroms; und

Feststellen, dass ein aktueller Betriebszustand des Hauptkörpers (10) ein Einsatzzustand ist, Steuern des Sauerstoff-Proportionalventils (40) so, dass es sich mit dem entsprechenden voreingestellten Öffnungsgrad öffnet, sowie Steuern des Gebläses (101) des Hauptkörpers (10) so, dass es mit der entsprechenden voreingestellten Drehzahl läuft, wenn der berechnete Luftstromdruck an der Patienten-Schnittstelle (203) einem ersten voreingestellten Druck-Zielwert nicht gleich ist.

12. Computerprogramm nach Anspruch 11, wobei der Schritt, in dem, wenn der berechnete Luftstromdruck an der Patienten-Schnittstelle (203) nicht gleich ist, festgestellt wird, dass ein aktueller Betriebszustand des Hauptkörpers (10) ein Einsatzzustand ist und das Sauerstoff-Proportionalventil (40) so gesteuert wird, dass es sich mit dem entsprechenden voreingestellten Öffnungsgrad öffnet, und das Gebläse (101) des Hauptkörpers (10) so gesteuert wird, dass es mit der entsprechenden voreingestellten Drehzahl läuft, umfasst:

Feststellen, dass der aktuelle Betriebszustand des Hauptkörpers (10) ein Einatmungs-Zustand ist, Steuern des Sauerstoff-Proportionalventils (40) so, dass es sich mit einem entsprechenden zweiten Öffnungsgrad öffnet, und Steuern des Gebläses (101) des Hauptkörpers (10) so, dass es mit einer entsprechenden zweiten voreingestellten Drehzahl läuft, wenn der berechnete Luftstromdruck an der Patienten-Schnittstelle (203) unter dem ersten voreingestellten Druck-Zielwert liegt; sowie

Feststellen, dass der aktuelle Betriebszustand des Hauptkörpers (10) ein Ausatmungs-Zustand ist, Steuern des Sauerstoff-Proportionalventils (40) so, dass es sich mit einem entsprechenden dritten Öffnungsgrad öffnet, und Steuern des Gebläses (101) des Hauptkörpers (10) so, dass es mit einer entsprechenden dritten voreingestellten Drehzahl läuft, wenn der berechnete Luftstromdruck an der Patienten-Schnittstelle (203) über dem ersten voreingestellten Druck-Zielwert liegt.

**13.** Computerprogramm nach Anspruch 11 oder 12, wobei das Verfahren des Weiteren umfasst:

Feststellen, dass der aktuelle Betriebszustand des Hauptkörpers (10) ein Ruhezustand ist, Steuern des Sauerstoff-Proportionalventils (40) so, dass es sich mit einem entsprechenden ersten Öffnungsgrad öffnet, und Steuern des Gebläses (101) des Hauptkörpers (10) so, dass es mit einer entsprechenden ersten voreingestellten Drehzahl läuft, wenn der berechnete Luftstromdruck an der Patienten-Schnittstelle (203) einem zweiten voreingestellten Druck-Zielwert gleich ist und über eine voreingestellte Zeit aufrechterhalten wird.

**14.** Computerprogramm nach Anspruch 13, wobei das Verfahren des Weiteren umfasst:

Feststellen, dass der aktuelle Betriebszustand des Hauptkörpers (10) ein Bereitschaftszustand ist, Steuern des Sauerstoff-Proportionalventils (40) so, dass es sich schließt, und Steuern eines Befeuchters (101) so, dass er startet, sowie Steuern des Gebläses (101) des Hauptkörpers (10) so, dass es mit einer entsprechenden vierten voreingestellten Drehzahl läuft; und

wobei die vierte voreingestellte Drehzahl niedriger ist als die erste voreingestellte Drehzahl.

**Revendications**

**1.** Appareil de thérapie par ventilation, comprenant : un corps principal (10), un tube respiratoire (20), une interface patient (203), un module d'alimentation d'oxygène (30), une vanne proportionnelle à oxygène (40) et un module de commande (50) ;

le corps principal (10) étant conçu pour délivrer un gaz avec une pression prédéfinie et un flux prédéfini, et le corps principal (10) comprenant une extrémité de sortie ;

le tube respiratoire (20) comprenant une première extrémité (201) et une seconde extrémité (202) qui communiquent l'une avec l'autre, et la première extrémité (201) du tube respiratoire (20) communiquant avec l'extrémité de sortie ;

la seconde extrémité (202) du tube respiratoire (20) étant raccordée à l'interface patient (203), l'interface patient (203) étant conçue pour être portée sur une cavité nasale d'un patient, et lorsque l'interface patient (203) est portée sur la cavité nasale du patient, un espace de sortie d'air étant disposé entre l'interface patient (203) et la cavité nasale du patient ;

le module d'alimentation d'oxygène (30) étant raccordé au corps principal (10) à travers la vanne proportionnelle à oxygène (40), et un ventilateur (101) étant disposé dans le corps principal (10) ;

le corps principal (10) étant conçu pour mélanger un air inhalé avec l'oxygène fourni par le module d'alimentation d'oxygène (30), et délivrer un mélange de gaz à travers le tube respiratoire (20) ;

le module de commande (50) étant raccordé au corps principal (10) et la vanne proportionnelle à oxygène (40), respectivement ; et

le module de commande (50) étant conçu pour détecter des paramètres de sortie du corps principal (10), les paramètres de sortie comprenant une pression d'écoulement d'air et un écoulement d'air au niveau de l'extrémité de sortie du corps principal (10),

lorsqu'il est déterminé que le corps principal (10) se trouve sous un état prédéfini, le module de commande (50), en fonction des paramètres de sortie, étant conçu pour :

commander à la vanne proportionnelle à oxygène (40) de s'ouvrir à un degré d'ouverture prédéfini correspondant, et

commander au ventilateur (101) du corps principal (10) de fonctionner à une vitesse de rotation prédéfinie correspondante.

**2.** Appareil de thérapie par ventilation selon la revendication 1,

le module de commande (50) étant conçu pour calculer une pression d'écoulement d'air au niveau de l'interface patient à travers la pression d'écoulement d'air et l'écoulement d'air ; et

lorsque la pression d'écoulement d'air calculée au niveau de l'interface patient (203) n'est pas égale à une première valeur de pression cible prédéfinie, il est déterminé qu'un état de travail présent du corps principal (10) est un état d'utilisation, le module de commande (50) étant conçu pour commander à la vanne proportionnelle à oxygène (40) de s'ouvrir au niveau du degré d'ouverture prédéfini correspondant, et pour commander au ventilateur (101) du corps principal (10) de fonctionner à la vitesse de rotation prédéfinie correspondante.

**3.** Appareil de thérapie par ventilation selon la revendication 2, l'opération que lorsque la pression d'écoulement d'air calculée au niveau de l'interface patient (203) n'est pas égale à la première valeur de pression cible prédéfinie, il est déterminé qu'un état de travail présent du corps principal (10) est un état d'utilisation, le module de commande (50) étant conçu pour commander à la vanne proportionnelle à oxygène (40) de s'ouvrir au degré d'ouverture prédéfini correspondant, et pour commander au ventilateur (101) du corps principal (10) de fonctionner à la vitesse de rotation prédéfinie correspondante, comprenant :

si la pression d'écoulement d'air calculée au niveau de l'interface patient (203) est inférieure à la première valeur de pression cible prédéfinie, il est déterminé que l'état de travail présent du corps principal (10) est un état inspiratoire, le module de commande (50) étant en outre conçu pour commander à la vanne proportionnelle à oxygène (40) de s'ouvrir à un second degré d'ouverture correspondant, et pour commander au ventilateur (101) du corps principal (10) de fonctionner à une seconde vitesse de rotation prédéfinie correspondante ; et

si la pression d'écoulement d'air calculée au niveau de l'interface patient (203) est supérieure à la première valeur de pression cible prédéfinie, il est déterminé que l'état de travail présent du corps principal (10) est un état d'expiration, le module de commande (50) étant en outre conçu pour commander à la vanne proportionnelle à oxygène (40) de s'ouvrir à un troisième degré d'ouverture correspondant, et pour commander au ventilateur (101) du corps principal (10) de fonctionner à une troisième vitesse de rotation prédéfinie correspondante.

**4.** Appareil de thérapie par ventilation selon la revendication 2 ou 3, lorsque la pression d'écoulement d'air calculée au niveau de l'interface patient (203) est égale à une seconde valeur de pression cible prédéfinie et est maintenue pour une durée prédéfinie, il est déterminé que l'état de travail présent du corps principal (10) est un état sans utilisation, le module de commande (50) étant en outre conçu pour commander à la vanne proportionnelle à oxygène (40) de s'ouvrir à un premier degré d'ouverture correspondant, et pour commander au ventilateur (101) du corps principal (10) de fonctionner à une première vitesse de rotation prédéfinie correspondante ;

la seconde valeur de pression cible prédéfinie étant inférieure à la première valeur de pression cible prédéfinie.

**5.** Appareil de thérapie par ventilation selon la revendication 4, le second degré d'ouverture étant plus grand que le premier degré d'ouverture, le premier degré d'ouverture étant supérieur ou égal au troisième degré d'ouverture ; la seconde vitesse de rotation prédéfinie étant supérieure à la première vitesse de rotation prédéfinie, et la première vitesse de rotation prédéfinie étant supérieure ou égale à la troisième vitesse de rotation prédéfinie.

**6.** Appareil de thérapie par ventilation selon l'une quelconque des revendications 1 à 5, l'appareil de thérapie par ventilation comprenant en outre :
un humidificateur, conçu pour chauffer et humidifier le gaz de sortie.

**7.** Appareil de thérapie par ventilation selon la revendication 6, lorsque la pression d'écoulement d'air calculée au niveau de l'interface patient (203) est égale à la seconde valeur de pression cible prédéfinie et est maintenue pour une durée prédéfinie, il est déterminé que l'état de travail présent du corps principal (10) est un état de veille, le module de commande (50) étant en outre conçu pour commander à la vanne proportionnelle à oxygène (40) de se fermer, et commander à l'humidificateur de démarrer, et pour commander au ventilateur (101) du corps principal (10) de fonctionner à une quatrième vitesse de rotation prédéfinie correspondante ;

la quatrième vitesse de rotation prédéfinie étant inférieure à la première vitesse de rotation prédéfinie.

**8.** Appareil de thérapie par ventilation selon l'une quelconque des revendications 1 à 7, l'opération que le module de commande (50) est conçu pour détecter des paramètres de sortie du corps principal (10), lorsqu'il est déterminé que le corps principal (10) se trouve sous un état prédéfini, le module de commande (50) commandant à la vanne proportionnelle à oxygène (40) de s'ouvrir à un degré d'ouverture prédéfini correspondant en fonction des paramètres de sortie, et commandant au ventilateur (101) du corps principal (10) de fonctionner à une vitesse de rotation

EP 3 871 720 B1

prédéfinie correspondante, comprenant :

le module de commande (50) est conçu pour détecter les paramètres de sortie du corps principal (10) ; et le module de commande (50) est en outre conçu pour surveiller une valeur de flux respiratoire du patient ;
lorsqu'il est déterminé que le corps principal (10) se trouve sous un état de priorité d'écoulement, le module de commande (50) est conçu pour ajuster les paramètres de sortie du corps principal (10) pour qu'ils soient supérieurs à la valeur de flux respiratoire du patient ; et
lorsqu'il est déterminé que le corps principal (10) se trouve sous un état de priorité de pression, le module de commande (50) est conçu pour ajuster les paramètres de sortie du corps principal (10) pour qu'ils soient d'une valeur de pression positive.

9. Appareil de thérapie par ventilation selon l'une quelconque des revendications 1 à 8, le tube respiratoire (20) et le corps principal (10) étant raccordés à travers un trajet de gaz et un circuit, et le circuit et le trajet de gaz se trouvant simultanément sur marche et arrêt.

10. Programme informatique comprenant un code lisible par ordinateur, lorsque le code lisible par ordinateur est exécuté sur un dispositif de traitement informatique d'un module de commande (50) d'un appareil de thérapie par ventilation selon l'une quelconque des revendications 1 à 9, amenant le dispositif de traitement informatique à exécuter un procédé de commande de l'appareil de thérapie par ventilation, le procédé consistant à :

détecter des paramètres de sortie du corps principal (10), les paramètres de sortie comprenant une pression d'écoulement d'air et un écoulement d'air à l'extrémité de sortie du corps principal (10) ;
lorsqu'il est déterminé que le corps principal (10) se trouve sous un état prédéfini, en fonction des paramètres de sortie,

commander à une vanne proportionnelle à oxygène (40) raccordée au corps principal (10) de s'ouvrir à un degré d'ouverture prédéfini correspondant, pour permettre à un module d'alimentation d'oxygène (30) d'alimenter de l'oxygène pour le corps principal (10) à travers la vanne proportionnelle à oxygène (40), et commander à un ventilateur (101) du corps principal (10) de fonctionner à une vitesse de rotation prédéfinie correspondante, pour permettre au corps principal (10) d'inspirer de l'air ;
mélanger l'air à inhaler par le corps principal (10) avec l'oxygène alimenté par le module d'alimentation d'oxygène (30), et obtenir un mélange de gaz ; et
à délivrer le mélange de gaz à travers un tube respiratoire (20).

11. Programme informatique selon la revendication 10,
l'étape dans laquelle il est déterminé que le corps principal (10) se trouve sous un état prédéfini, de commande d'une vanne proportionnelle à oxygène (40) raccordée au corps principal (10) de s'ouvrir à un degré d'ouverture prédéfini correspondant en fonction des paramètres de sortie, pour permettre à un module d'alimentation d'oxygène (30) d'alimenter de l'oxygène pour le corps principal (10) à travers la vanne proportionnelle à oxygène (40), et de commander à un ventilateur (101) du corps principal (10) de fonctionner à une vitesse de rotation prédéfinie correspondante, pour permettre au corps principal (10) d'inspirer de l'air consistant à :

calculer une pression d'écoulement d'air au niveau d'une interface patient (203) à travers la pression d'écoulement d'air et l'écoulement d'air ; et
lorsque la pression d'écoulement d'air calculée au niveau de l'interface patient (203) n'est pas égale à une première valeur de pression cible prédéfinie, à déterminer qu'un état de travail présent du corps principal (10) est un état d'utilisation, à commander à la vanne proportionnelle à oxygène (40) de s'ouvrir au degré d'ouverture prédéfini correspondant, et à commander au ventilateur (101) du corps principal (10) de fonctionner à la vitesse de rotation prédéfinie correspondante.

12. Programme informatique selon la revendication 11,
l'étape dans laquelle la pression d'écoulement d'air calculée au niveau de l'interface patient (203) n'est pas égale à une première valeur de pression cible prédéfinie, de détermination qu'un état de travail présent du corps principal (10) est un état d'utilisation, de commande à la vanne proportionnelle à oxygène (40) de s'ouvrir au degré d'ouverture prédéfini correspondant, et de commande au ventilateur (101) du corps principal (10) de fonctionner à la vitesse de rotation prédéfinie correspondante, consistant à :

si la pression d'écoulement d'air calculée au niveau de l'interface patient (203) est inférieure à la première valeur de pression cible prédéfinie, à déterminer que l'état de travail présent du corps principal (10) est un état inspiratoire, à commander à la vanne proportionnelle à oxygène (40) de s'ouvrir à un second degré d'ouverture correspondant, et à commander au ventilateur (101) du corps principal (10) de fonctionner à une seconde vitesse de rotation prédéfinie correspondante ; et

si la pression d'écoulement d'air calculée au niveau de l'interface patient (203) est supérieure à la première valeur de pression cible prédéfinie, à déterminer que l'état de travail présent du corps principal (10) est un état d'expiration, à commander à la vanne proportionnelle à oxygène (40) de s'ouvrir à un troisième degré d'ouverture correspondant, et à commander au ventilateur (101) du corps principal (10) de fonctionner à une troisième vitesse de rotation prédéfinie correspondante.

13. Programme informatique selon la revendication 11 ou 12, le procédé consistant en outre à :

lorsque la pression d'écoulement d'air calculée au niveau de l'interface patient (203) est égale à une seconde valeur de pression cible prédéfinie, et est maintenue pour une durée prédéfinie, déterminer que l'état de travail présent du corps principal (10) est un état sans utilisation, à commander à la vanne proportionnelle à oxygène (40) de s'ouvrir à un premier degré d'ouverture correspondant, et à commander au ventilateur (101) du corps principal (10) de fonctionner à une première vitesse de rotation prédéfinie correspondante ;
la seconde valeur de pression cible prédéfinie étant inférieure à la première valeur de pression cible prédéfinie.

14. Programme informatique selon la revendication 13, le procédé consistant en outre à :

lorsque la pression d'écoulement d'air calculée au niveau de l'interface patient (203) est égale à la seconde valeur de pression cible prédéfinie, et est maintenue pour la durée prédéfinie, déterminer que l'état de travail présent du corps principal (10) est un état de veille, à commander à la vanne proportionnelle à oxygène (40) de se fermer, et à commander à un humidificateur de démarrer, et à commander au ventilateur (101) du corps principal (10) de fonctionner à une quatrième vitesse de rotation prédéfinie correspondante ; et
la quatrième vitesse de rotation prédéfinie étant inférieure à la première vitesse de rotation prédéfinie.

Fig. 1

Fig.2

Fig.3

Fig. 4

Patient respiratory flow curve
Ventilator output flow curve
Ventilator output Oxygen flow curve

Fig.5

Pressure

——— Patient respiratory pressure curve

- - - - Ventilator output pressure curve

Time

Patient's exhaling

Patient's inhaling

Fig. 6

| detecting output parameters of a main body | 701 |

| when it is determined that the main body is in a preset state, controlling an oxygen proportional valve connected to the main body to open at a corresponding preset opening degree according to the output parameters, to allow an oxygen supply module to supply oxygen for the main body through the oxygen proportional valve, and controlling a fan of the main body to run at a corresponding preset rotating speed, to allow the main body to inhale air | 702 |

| mixing the air inhaled by the main body with the oxygen supplied by the oxygen supply module, and obtaining a gas mixture | 703 |

| outputting the gas mixture through a respiratory pipe | 704 |

Fig. 7

detecting output parameters of a main body | 801

calculating an airflow pressure at a patient interface through the airflow pressure and the air flow | 802

when the airflow pressure at the patient interface is not equal to a first preset target pressure value, determining that a current working state of the main body is a use state, controlling the oxygen proportional valve to open at the corresponding preset opening degree, and controlling the fan of the main body to run at the corresponding preset rotating speed | 803

mixing the air inhaled by the main body with the oxygen supplied by the oxygen supply module, and obtaining a gas mixture | 804

outputting the gas mixture through a respiratory pipe | 805

Fig. 8

Processor
1010

Memory 1020

Space of program codes 1030

Program codes for performing steps of
the method according to the disclosure

Computing
processing device

Fig. 9

Storage unit of program codes

1031'

Readable codes for performing steps of the
method according to the disclosure

Fig.10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201811261598
- US 2016287824 A1
- KR 20110094649 A
- WO 2017055995 A1